(19) Europäisches Patentamt
European Patent Office
Office européen des brevets

(11) EP 2 450 335 B1

(12) EUROPEAN PATENT SPECIFICATION

(45) Date of publication and mention of the grant of the patent:
**25.03.2015 Bulletin 2015/13**

(21) Application number: **10794053.8**

(22) Date of filing: **24.06.2010**

(51) Int Cl.:
***C07C 41/30*** (2006.01) ***B01J 23/89*** (2006.01)
***C07C 43/205*** (2006.01) ***C07B 61/00*** (2006.01)

(86) International application number:
**PCT/JP2010/060721**

(87) International publication number:
**WO 2011/001884 (06.01.2011 Gazette 2011/01)**

# (54) METHOD FOR SYNTHESIZING COMPOUND AND CATALYST FOR SYNTHESIS REACTION

VERFAHREN ZUR SYNTHESE EINER VERBINDUNG UND KATALYSATOR FÜR DIE SYNTHESEREAKTION

PROCÉDÉ DE SYNTHÈSE D'UN COMPOSÉ ET CATALYSEUR POUR UNE RÉACTION DE SYNTHÈSE

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**

(30) Priority: **30.06.2009 JP 2009156259**

(43) Date of publication of application:
**09.05.2012 Bulletin 2012/19**

(73) Proprietors:
- **Hokko Chemical Industry Co., Ltd.**
  **Chuo-ku, Tokyo 103-8341 (JP)**
- **DAIHATSU MOTOR COMPANY, LTD.**
  **Ikeda-shi**
  **Osaka 563-8651 (JP)**

(72) Inventors:
- **KANEKO, Kimiyoshi**
  **Tokyo 103-8341 (JP)**
- **TANAKA, Hirohisa**
  **Gamo-gun**
  **Shiga 520-2593 (JP)**

(74) Representative: **Schwabe - Sandmair - Marx**
**Patentanwälte**
**Stuntzstraße 16**
**81677 München (DE)**

(56) References cited:
**WO-A1-2005/090238** **WO-A1-2005/105719**
**WO-A1-2006/098396** **WO-A1-2006/098398**
**JP-A- 2 265 648** **JP-A- 8 064 879**
**US-A1- 2005 215 804**

- **ANDREWS S P ET AL: "Heterogeneous or Homogeneous? A case study involving Palladium-containing perovskites in the Suzuki Reaction", ADVANCED SYNTHESIS AND CATALYSIS, WILEY, WEINHEIM, DE, vol. 347, 8 July 2005 (2005-07-08), pages 647-654, XP003001250, ISSN: 1615-4169, DOI: 10.1002/ADSC.200404331**
- **JETI vol. 54, no. 10, 01 September 2006, pages 49 - 52, XP008149211**
- **STUDIES IN SURFACE SCIENCE AND CATALYSIS vol. 116, 1998, pages 581 - 589, XP008149406**
- **MATERIALS SCIENCE FORUM vol. 228-231, 1996, pages 795 - 799, XP008149212**
- **VENTE J F, BATTLE P D: 'Control of magnetism by crystal chemistry in T'-phase R2Cu1-xPdxO4 (R=Nd, Sm, Eu, Gd; 0< x< 0.2)' PHYSICAL REVIEW B: CONDENSED MATTER AND MATERIALS PHYSICS vol. 58, no. 5, 1998, pages 2699 - 2707, XP008149415**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 450 335 B1

## Description

### Technical Field

The present invention relates to a method for synthesizing a compound and a catalyst for synthesis reaction and, more particularly, to a method for synthesizing a compound through Suzuki Cross-Couplings and a catalyst for synthesis reaction used in the synthesizing method.

### Background Art

In recent years, a catalyst composed of a palladium-containing perovskite-type composite oxide which is highly active as a catalyst for Suzuki Cross-Couplings and is also capable of being recovered and reused after the completion of the reaction has attracted attention.

There has been proposed, for example, a catalyst composed of a palladium-containing perovskite-type composite oxide represented in the general formula of $AB_{1-X}Pd_XO_3$ (see the following Patent Documents 1 and 2

### Prior Art Document

### Patent Document

Patent Document 1: Japanese Unexamined Patent Publication No. 2005-314355
Patent Document 2: US 2005/0215804 A1

### Summary of the Invention

### Problems to be solved by the Invention

In Suzuki Cross-Couplings, palladium which shows activity of a catalyst is expensive and therefore, it is strongly required that in order to reduce the production cost, the effect thereof is effectively developed in an amount as small as possible by increasing mole number (turnover number) of a reaction product per mol palladium on the industrial basis.

An object of the present invention is to provide a catalyst for synthesis reaction that is capable of effectively developing an activity of palladium and synthesizing a compound less expensively, and a method for synthesizing a compound in which the catalyst for synthesis reaction is used.

### Solution to the Problems

To achieve the above-described object, the method for synthesizing a compound of the present invention includes reacting a compound represented by the following general formula (2) with a compound represented by the following general formula (3) in the presence of a palladium-containing layered perovskite-type composite oxide represented by the following general formula (1):

$$Ln_2M_yCu_{l-x-y}Pd_xO_{4\pm\delta} \qquad (1)$$

wherein Ln represents elements composed of at least one essential element selected from La, Pr, Nd, Sm, Eu, and Gd and at least one optional element selected from Y, Ce, Yb, Ca, Sr, and Ba; M represents at least one element selected from Cr, Mn, Fe, Co, Ni, and A1; x, indicating an atomic proportion, is $0.001 \leq x \leq 0.4$; y, indicating an atomic proportion, is $0 \leq y \leq 0.5$; and $\delta$ indicates an oxygen excess amount or an oxygen deficiency amount,

$$R_1\text{-}X \qquad (2)$$

wherein $R_1$ represents an aryl group which may have a substituent, a heterocyclic group which may have a substituent, or an alkenyl group which may have a substituent; and X represents a halogen atom, a trifluoromethanesulfonyloxy group, a p-toluenesulfonyloxy group or a methanesulfonyloxy group, and

$$R_2\text{-}B(ORa)_2 \qquad (3)$$

wherein $R_2$ represents an aryl group which may have a substituent, a heterocyclic group which may have a substituent, or an alkenyl group which may have a substituent; and Ra represents a hydrogen atom or an alkyl group which may have a substituent. Instead of Ra, a ring including -OBO- may be formed through an arylene group which may have a substituent or an alkylene group which may have a substituent, both of which serve as a bond of -OBO-.

In the method for synthesizing a compound of the present invention, it is preferable that in the above-described general formula (1), Ln is at least one element selected from La, Nd, and Gd.

In the method for synthesizing a compound of the present invention, it is preferable that in the above-described general formula, x represents an atomic proportion satisfying the following relation: $0.005 \leq x \leq 0.05$.

In the method for synthesizing a compound of the present invention, it is preferable that a compound represented by the above-described general formula (2) is reacted with a compound represented by the above-described general formula (3) by using a reaction solvent containing a glycol ether.

The catalyst for synthesis reaction of the present invention includes a palladium-containing layered perovskite-type composite oxide which is represented by the following general formula (1), and is used so as to react a compound represented by the following general formula (2) with a compound represented by the following general formula (3):

$$Ln_2M_yCu_{1-x-y}Pd_xO_{4\pm\delta} \qquad (1)$$

wherein Ln represents elements composed of at least one essential element selected from La, Pr, Nd, Sm, Eu, and Gd and at least one optional element selected from Y, Ce, Yb, Ca, Sr, and Ba; M represents at least one element selected from Cr, Mn, Fe, Co, Ni, and Al; x, indicating an atomic proportion, is $0{,}001 \leq x \leq 0.4$; y, indicating an atomic proportion, is $0 \leq y \leq 0.5$; and $\delta$ indicates an oxygen excess amount or an oxygen deficiency amount,

$$R_1\text{-}X \qquad (2)$$

wherein $R_1$ represents an aryl group which may have a substituent, a heterocyclic group which may have a substituent, or an alkenyl group which may have a substituent; and X represents a halogen atom, a trifluoromethanesulfonyloxy group, a p-toluenesulfonyloxy group or a methanesulfonyloxy group, and

$$R_2\text{-}B(ORa)_2 \qquad (3)$$

wherein $R_2$ represents an aryl group which may have a substituent, a heterocyclic group which may have a substituent, or an alkenyl group which may have a substituent; and Ra represents a hydrogen atom or an alkyl group which may have a substituent. Instead of Ra, a ring including -OBO- may be formed through an arylene group which may have a substituent or an alkylene group which may have a substituent, both of which serve as a bond of-OBO-.

Also, in the catalyst for synthesis reaction of the present invention, it is preferable that in the above-described general formula (1), Ln is at least one element selected from La, Nd, and Gd.

Further, in the catalyst for synthesis reaction of the present invention, it is preferable that in the above-described general formula, x represents an atomic proportion satisfying the following relation: $0.005 \leq x \leq 0.05$.

## Effect of the Invention

In the method for synthesizing a compound of the present invention, a catalyst for synthesis reaction composed of a palladium-containing layered perovskite-type composite oxide represented by the general formula of $Ln_2M_yCu_{1-x-y}Pd_xO_{4\pm\delta}$ is used, so that by increasing the turnover number of a catalyst, even when a small amount of palladium is used, an activity of palladium can be effectively developed. Thus, a compound can be synthesized less expensively.

In the catalyst for synthesis reaction of the present invention, in Suzuki Cross-Couplings, by increasing the turnover number thereof, even when a small amount of palladium is used, an activity of palladium can be effectively developed. Thus, a compound can be synthesized less expensively. Therefore, the catalyst for synthesis reaction of the present invention can be effectively used as a catalyst for synthesis reaction in Suzuki Cross-Couplings.

## Brief Description of the Drawings

FIG. 1 shows a spectrum diagram of an X-ray diffraction of powder in Production Example 1.
FIG. 2 shows a spectrum diagram of an X-ray diffraction of powder in Production Example 2.
FIG. 3 shows a spectrum diagram of an X-ray diffraction of powder in Production Example 3.

FIG. 4 shows a spectrum diagram of an X-ray diffraction of powder in Production Example 4,
FIG. 5 shows a spectrum diagram of an X-ray diffraction of powder in Production Example 5.
FIG. 6 shows a spectrum diagram of an X-ray diffraction of powder in Production Example 6.
FIG. 7 shows a spectrum diagram of an X-ray diffraction of powder in Production Example 7.
FIG. 8 shows a spectrum diagram of an X-ray diffraction of powder in Production Example 8.
FIG. 9 shows a spectrum diagram of an X-ray diffraction of powder in Production Example 9.
FIG. 10 shows a spectrum diagram of an X-ray diffraction of powder in Production Example 10.
FIG. 11 shows a spectrum diagram of an X-ray diffraction of powder in Production Example 11.
FIG. 12 shows a spectrum diagram of an X-ray diffraction of powder in Production Example 12.
FIG. 13 shows a spectrum diagram of an X-ray diffraction of powder in Production Example 13.
FIG. 14 shows a spectrum diagram of an X-ray diffraction of powder in Production Example 14.
FIG. 15 shows a spectrum diagram of an X-ray diffraction of powder in Production Example 15.
FIG. 16shows a spectrum diagram of an X-ray diffraction of powder in Production Example 16.
FIG. 17 shows a spectrum diagram of an X-ray diffraction of powder in Production Example 17.

Embodiment of the Invention

The method for synthesizing a compound of the present invention is carried out in the presence of a layered perovskite-type composite oxide represented by the general formula of $A_2BO_4$.

In the present invention, the layered perovskite-type composite oxide contains palladium and is represented by the following general formula (1):

$$Ln_2M_yCu_{l\text{-}x\text{-}y}Pd_xO_{4\pm\delta} \qquad (1)$$

wherein Ln represents elements composed of at least one essential element selected from La, Pr, Nd, Sm, Eu, and Gd and at least one optional element selected from Y, Ce, Yb, Ca, Sr, and Ba; M represents at least one element selected from Cr, Mn, Fe, Co, Ni, and Al; x, indicating an atomic proportion, is $0.001 \leq x \leq 0.4$; y, indicating an atomic proportion, is: $0 \leq y \leq 0.5$; and $\delta$ indicates an oxygen excess amount or an oxygen deficiency amount.

In the general formula (1), Ln inevitably contains at least one essential element selected from La, Pr, Nd, Sm, Eu, and Gd. Preferably, Ln contains at least one element selected from La, Nd, and Gd. These elements can be used alone or in combination.

Also, in the general formula (1), Ln may or may not contain at least one optional element selected from Y, Ce, Yb, Ca, Sr, and Ba.

When the above-described optional element is contained, for example, the layered perovskite-type composite oxide of the present invention can be represented, for example, by the following general formula (1') by defining the above-described essential element as Ln and optional element as Ln'.

$$(Ln_{1\text{-}z}Ln'_z)M_yCu_{l\text{-}x\text{-}y}Pd_xO_{4\pm\delta} \qquad (1')$$

In the general formula (1'), z indicates an atomic proportion of the optional element Ln', for example, satisfying the following relation: $0.01 \leq z \leq 0.5$, and preferably $0.1 \leq z \leq 0.5$. That is, when the optional element Ln' is contained, preferably, the atomic proportion thereof is 0.5 or less.

In the general formula (1), an atomic proportion y of M satisfies the following relation: $0 \leq y \leq 0.5$. That is, M is an optional element and may or may not be contained. When M is contained, the atomic proportion thereof is 0.5 or less.

In the general formula (1), an atomic proportion x of Pd satisfies the following relation: $0.001 \leq x \leq 0.4$, that is, x is 0.4 or less. Preferably, x satisfies the following relation: $0.005 \leq x \leq 0.05$. When the atomic proportion of Pd exceeds 0.4, it may be difficult to dissolve Pd to form a solid solution and further, the cost is inevitably increased. When the atomic propotion x of Pd satisfies the following relation: $0.005 \leq x \leq 0.05$, the turnover number of a catalyst can further be increased.

In the general formula (1), Cu is contained in the layered perovskite-type composite oxide with an atomic proportion satisfying the remainders (1-x-y) of M and Pd.

In the general formula (1), $\pm\delta$ represents an oxygen excess amount or an oxygen deficiency amount, and more specifically, it represents an excessive atomic proportion or a deficient atomic proportion of oxygen caused by allowing the elements coordinated on the site A to be excessive or deficient as compared with A:B:O = 2:1:4, which is a theoretical constituent ratio of the layered perovskite-type composite oxide.

In the general formula (1), a ratio of the elements coordinated on the site A with respect to the elements coordinated on the site B (an atomic proportion of A/B = Ln / (an atomic proportion of M + an atomic proportion of Cu + an atomic proportion of Pd)) indicates A/B = 2 in the theoretical constituent ratio. However, the ratio changes due to the excess or deficiency of the elements coordinated on the site A and preferably, satisfies the following relation: $1.80 \leq A/B$

≤ 2.20. When A/B is within this range, crystal collapse of the layered perovskite-type composite oxide can be prevented and an excellent layered crystal state thereof can be maintained.

In the present invention, a specific surface area of the layered perovskite-type composite oxide containing palladium is, for example, 20 $m^2/g$ or less, and preferably, 12 $m^2/g$ or less. A specific surface area can be calculated by a BET method.

The layered perovskite-type composite oxide containing palladium can be prepared according to any suitable procedure for the preparation of composite oxide without any specific limitations. Examples thereof include an alkoxide method, coprecipitation method, and citrate complex method.

In the alkoxide method, for example, an alkoxide mixed solution is prepared, which contains alkoxides of the above-described respective elements excluding Pd in the above-described stoichiometric ratio. The alkoxide mixed solution is precipitated on hydrolysis by adding an aqueous solution containing salts of Pd thereto. The resulting precipitate is dried and then subjected to a heat treatment.

Examples of the alkoxides of the respective elements include alcoholates each formed from the respective elements and an alkoxy group such as methoxy, ethoxy, propoxy, isopropoxy, or butoxy; and alkoxyalcoholates of the respective elements represented by the following general formula (4):

$$E[OCH(R_3)\text{-}(CH_2)_i\text{-}OR_4]_j \qquad (4)$$

wherein E represents the respective elements; $R_3$ represents a hydrogen atom or an alkyl group having 1 to 4 carbon atoms; $R_4$ represents an alkyl group having 1 to 4 carbon atoms; i represents an integer of 1 to 3; and j represents an integer of 2 to 3.

More specific examples of the alkoxyalcoholates include methoxyethylate, methoxypropylate, methoxybutylate, ethoxyethylate, ethoxypropylate, propoxyethylate, and butoxyethylate.

The alkoxide mixed solution is prepared, for example, by adding an alkoxide of the respective elements to an organic solvent in an amount that establishes the above-described stoichiometric ratio and mixing them with stirring.

The organic solvent is not specifically limited, as long as it can dissolve an alkoxide of the respective elements. Examples of such organic solvents include aromatic hydrocarbons, aliphatic hydrocarbons, alcohols, ketones, and esters. Preferably, aromatic hydrocarbons such as benzene, toluene, and xylene are used.

Then, the alkoxide mixed solution is precipitated by adding an aqueous solution containing salts of Pd in a predetermined stoichiometric ratio. Examples of the aqueous solution containing salts of Pd include an aqueous solution of nitrate, aqueous solution of chloride, aqueous solution of hexaammine chloride, aqueous solution of dinitrodiammine nitrate, aqueous solution of hexachloro acid hydrate, and potassium cyanide salt.

The resulting precipitate is dried, for example, by vacuum drying or forced-air drying and is subjected to a primary heat treatment at about 400 to 1,000°C, and preferably at about 500 to 850°C, and furthermore, followed by a secondary heat treatment at about 700 to 1,100°C, if necessary. Thus, a layered perovskite-type composite oxide can be prepared.

In such an alkoxide method, the composite oxide may be alternatively prepared in the following manner. A solution containing organometallic salts of Pd is added to the above-described alkoxide mixed solution to prepare a homogenous mixed solution. The homogenous mixed solution is precipitated by adding water thereto. The resulting precipitate is dried and then subjected to a heat treatment.

Examples of the organometallic salts of Pd include: carboxylate of Pd, which is derived from acetate, propionate or the like; and metal chelate complexes of Pd such as diketone complexes of Pd, which is derived from diketone compounds represented by the following general formula (5) or (6):

$$R_5COCHR_7COR_6 \qquad (5)$$

wherein $R_5$ represents an alkyl group having 1 to 4 carbon atoms, a fluoroalkyl group having 1 to 4 carbon atoms or an aryl group; $R_6$ represents an alkyl group having 1 to 4 carbon atoms, a fluoroalkyl group having 1 to 4 carbon atoms, an aryl group or an alkyloxy group having 1 to 4 carbon atoms; and $R_7$ represents a hydrogen atom or an alkyl group having 1 to 4 carbon atoms; and

$$CH_3CH(CORg)_2 \qquad (6)$$

wherein $R_8$ represents a hydrogen atom or an alkyl group having 1 to 4 carbon atoms.

In the above-described general formulas (5) and (6), examples of the alkyl group having 1 to 4 carbon atoms represented by $R_5$, $R_6$, $R_7$, and $R_8$ include methyl, ethyl, propyl, isopropyl, n-butyl, s-butyl, and t-butyl. Examples of the fluoroalkyl group having 1 to 4 carbon atoms represented by $R_5$ and $R_6$ include trifluoromethyl. Examples of the aryl group represented by $R_5$ and $R_6$ include phenyl. Examples of the alkyloxy group having 1 to 4 carbon atoms represented

by $R_6$ include methoxy, ethoxy, propoxy, isopropoxy, n-butoxy, s-butoxy, and t-butoxy.

Specific examples of the diketone compound include 2,4-pentanedione, 2,4-hexanedione, 2,2-dimethyl-3,5-hexanedione, 1-phenyl-1,3-butanedione, 1-trifluoromethyl-1,3-butanedione, hexafluoroacetylacetone, 1,3-diphenyl-1,3-propanedione, dipivaloylmethane, methyl acetoacetate, ethyl acetoacetate, and t-butyl acetoacetate.

The solution containing the organometallic salts of Pd can be prepared, for example, by adding an organometallic salt of Pd to an organic solvent in an amount that establishes the above-described stoichiometric ratio, and mixing them with stirring. The organic solvent can be any of the above-described organic solvents.

Thereafter, the prepared solution containing organometallic salts of Pd is mixed with the above-described alkoxide mixed solution, and the resulting homogenous mixed solution is precipitated by adding water thereto. The resulting precipitate is dried by vacuum drying or forced-air drying, for example, and is subjected to a primary heat treatment at about 400 to 1,000°C, and preferably at about 500 to 850°C, and furthermore, followed by a secondary heat treatment at about 700 to 1,100°C, if necessary. Thus, a layered perovskite-type composite oxide can be prepared.

In the coprecipitation method, for example, an aqueous mixed salt solution is prepared, which contains salts of the above-described respective elements in a predetermined stoichiometric ratio, a neutralizing agent is added to the aqueous mixed salt solution and/or the aqueous mixed salt solution is added to the neutralizing agent for coprecipitation thereof, and the resulting coprecipitate is dried and subjected to a heat treatment.

Examples of the salts of the respective elements include inorganic salts such as sulfates, nitrates, chlorides, and phosphates; and organic salts such as acetates and oxalates. The aqueous mixed salt solution can be prepared, for example, by adding the salts of the respective elements to water so as to establish a predetermined stoichiometric ratio and mixing them with stirring.

Then, the aqueous mixed salt solution is coprecipitated by adding the neutralizing agent thereto. Alternatively, a coprecipitate can be obtained by adding dropwise the aqueous mixed salt solution to an aqueous solution containing an excessive amount of neutralizing agent. The neutralizing agent includes, for example, ammonia; an organic base including amines such as triethylamine and pyridine; and an inorganic base such as sodium hydroxide, potassium hydroxide, potassium carbonate, and ammonium carbonate. The neutralizing agent is added in an amount such that the pH of the resulting solution after the addition of the neutralizing agent is adjusted within a range about from 6 to 14, and preferably about from 8 to 12.

The resulting coprecipitate is washed with water, if necessary, dried by vacuum drying or forced-air drying, and subjected to a primary heat treatment at about 400 to 1,000°C, and preferably at about 600 to 950°C, and furthermore subjected to a secondary heat treatment at about 900 to 1,100°C, if necessary. Thus, a layered perovskite-type composite oxide can be prepared.

In the citrate complex method, for example, an aqueous solution of a citrate salt mixture is prepared by mixing citric acid and salts of the above-described respective elements in an amount establishing a predetermined stoichiometric ratio. The aqueous solution of the citrate salt mixture is thoroughly dried to form a citrate complex of the above-described respective elements. The resulting citrate complex is provisionally baked and then subjected to a heat treatment.

Examples of the salts of the respective elements include the salts of the same kinds as those described above. The aqueous solution of the citrate salt mixture is, for example, prepared by preparing an aqueous mixed salt solution in the same manner as described above and adding an aqueous solution of citric acid to the aqueous mixed salt solution.

Then, the aqueous solution of the citrate salt mixture is thoroughly dried to form a citrate complex of the above-described respective elements. The drying process is carried out at a temperature at which the formed citrate complex is not decomposed, for example, from room temperature to about 150°C to remove water swiftly. Consequently, the above-described citrate complex of the respective elements can be formed.

The resulting citrate complex is then provisionally baked and heat treated. The provisional baking may be, for example, carried out at a temperature of 250°C or more in vacuum or in an inert atmosphere. Then, the provisionally baked substance is subjected to a primary heat treatment, for example, at about 300 to 1,000°C, and preferably at about 600 to 950°C, and furthermore, followed by a secondary heat treatment at about 900 to 1,100°C, if necessary, to obtain a layered perovskite-type composite oxide.

Further, palladium may be further supported on the above-described layered perovskite-type composite oxide containing palladium. In order to further support palladium on the layered perovskite-type composite oxide containing palladium, known methods can be applied without any specific limitations. For example, the composite oxide may be prepared in the following manner. A solution containing salts including palladium is prepared, the solution is impregnated in a layered perovskite-type composite oxide containing palladium, and baked. In this case, a supporting amount of palladium based on the layered perovskite-type composite oxide containing palladium is, for example, 10 parts by weight or less, and preferably from 0.1 to 5 parts by weight based on 100 parts by weight of the layered perovskite-type composite oxide containing palladium.

More specific examples of the layered perovskite-type composite oxide containing palladium include $La_2Cu_{0.95}Pd_{00.5}O_4$, $Pr_2Cu_{0.95}Pd_{0.05}O_4$, $Nd_2Cu_{0.6}Pd_{0.4}O_4$, $Nd_2Cu_{0.8}Pd_{0.2}O4$, $Nd_2Cu_{0.95}Pd_{0.5}O_4$, $Nd_2Cu_{0.99}Pd_{0.0}O_4$, $Nd_2Cu_{0.99}Pd_{0.0005}O_4$, $Nd_2Cu_{0.999}Pd0.001O_4$, $Gd_2Cu_{0.95}Pd_{0.5}O_4$, $Gd_2Cu_{0.94}Pd_{0.01}O_{3.95}$, $Gd_2Cu_{0.995}Pd_{0.005}O_4$,

$(La_{0.6}Sr_{0.4})_2Cu_{0.95}Pd_{0.05}O_4$, $(La_{0.6}Sr_{0.4})_2Cu_{0.94}Pd_{0.01}O_{3.95}$, $(Gd_{0.6}Sr_{0.4})_2CU_{0.94}Pd_{0.01}O_{3.95}$, $(Nd_{0.92}Ce_{0.08})_2Cu_{0.95}Pd_{0.05}O_4$, and $(La_{0.5}Y_{0.5})_2Cu_{0.95}P_{0.05}O_4$.

In the method for synthesizing a compound of the present invention, a compound represented by the following general formula (2) is reacted with a compound represented by the following general formula (3) in the presence of the above-described layered perovskite-type composite oxide containing palladium:

$R_1$-X (2)

wherein $R_1$ represents an aryl group which may have a substituent, a heterocyclic group which may have a substituent, or an alkenyl group which may have a substituent; and X represents a halogen atom, a trifluoromethanesulfonyloxy group, a p-toluenesulfonyloxy group or a methanesulfonyloxy group,

$R_2$-B(ORa)$_2$ (3)

wherein $R_2$ represents an aryl group which may have a substituent, a heterocyclic group which may have a substituent, or an alkenyl group which may have a substituent and Ra represents a hydrogen atom or an alkyl group which may have a substituent, and in stead of Ra, a ring including -OBO- may be formed through an arylene group which may have a substituent or an alkylene group which may have a substituent, both of which serve as a bond of-OBO-.

Examples of an aryl group of the aryl group which may have a substituent, which is represented by $R_1$ in the general formula (2) and $R_2$ in the general formula (3), include aryl groups having 6 to 14 carbon atoms such as phenyl, tolyl, xylyl, biphenyl, naphthyl, anthryl, phenanthryl, and azulenyl.

The substituent of the aryl group is not specifically limited, and examples of the substituent include such as hydrocarbon groups and hetero atom-containing hydrocarbon groups according to the purposes and applications. Examples thereof include alkyl groups having 1 to 4 carbon atoms such as methyl, ethyl, propyl, isopropyl, butyl, isobutyl, sec-butyl and tert-butyl; allenyl groups having 2 to 4 carbon atoms such as vinyl, 1-methylvinyl, 1-propenyl and allyl; alkynyl groups having 2 to 4 carbon atoms such as ethynyl, 1-propynyl and 1-propargyl; cycloalkyl groups having 3 to 6 carbon atoms such as cyclopropyl, cyclobutyl, cyclopentyl and cyclohexyl; cycloalkenyl groups having 5 to 7 carbon atoms such as cyclopentenyl and cyclohexenyl; aralkyl groups having 7 to 11 carbon atoms such as benzyl, $\alpha$-methylbenzyl and phenethyl; phenyl group; alkoxy groups having 1 to 6 carbon atoms such as methoxy, ethoxy, propoxy, isopropoxy, n-butoxy, iso-butoxy, sec-butoxy and tert-butoxy; phenoxy group; alkanoyl groups having 1 to 6 carbon atoms such as formyl, acetyl, propionyl, n-butyryl and iso-butyryl; benzoyl group; alkanoyloxy groups having 1 to 6 carbon atoms such as formyloxy, acetyloxy, propionyloxy, n-butyryloxy and iso-butyryloxy; benzoyloxy group; carboxyl group; alkoxycarbonyl groups having 2 to 7 carbon atoms such as methoxycarbonyl, ethoxycarbonyl, n-propoxycarbonyl, isopropoxycarbonyl, n-butoxycarbonyl, isobutoxycarbonyl and tert-butoxycarbonyl; carbamoyl groups; N-mono-$C_{1-4}$ alkylcarbamoyl groups such as N-methylcarbamoyl, N-ethylcarbamoyl, N-propylcarbamoyl, N-isopropylcarbamoyl and N-butylcarbamoyl; N,N-di-$C_{1-4}$ alkylcarbamoyl groups such as N,N-dimethylcarbamoyl, N,N-diethylcarbamoyl, N,N-dipropylcarbamoyl and N,N-dibutylcarbamoyl; cyclic aminocarbonyl groups such as 1-aziridinylcarbonyl, 1-azetidinylcarbonyl, 1-pyrolidinylcarbonyl, 1-piperidinylcarbonyl, N-methylpiperadinylcarbonyl and morpholinocarbonyl; halogen atoms such as fluorine, chlorine, bromine and iodine; mono-, di- or tri-halogeno-$C_{1-4}$ alkyl groups such as chloromethyl, dichloromethyl, trifluoromethyl and trifluoroethyl; oxo group; amidino group; imino group; amino group; mono-$C_{1-4}$ alkylamino groups such as methylamino, ethylamino, propylamino, isopropylamino and butylamino; di-$C_{1-4}$ alkylamino groups such as dimethylamino, diethylamino, dipropylamino, diisopropylamino and dibutylamino; 3 to 6-membered cyclic amino groups containing carbon atoms, a nitrogen atom and optionally 1 to 3 hetero atoms selected from oxygen atom, sulfur atom, nitrogen atom and the like, such as aziridinyl, azetidinyl, pyrolidinyl, pyrolinyl, pyrolyl, imidazolyl, pyrazolyl, imidazolydinyl, piperidino, morpholino, dihydropyridyl, pyridyl, N-methylpiperadinyl and N-ethylpiperadinyl; alkanoyl amido groups having 1 to 6 carbon atoms such as formamide, acetamide, trifluoroacetamide, propionylamide, butyrylamide and isobutyrylamide; benzamide group; carbamoylamino group; N-$C_{1-4}$ alkylcarbamoylamino groups such as N-methylcarbamoylamino, N-ethylcarbamoylamino, N-propylcarbamoylamino, N-isopropylcarbamoylamino and N-butylcarbamoylamino; N,N-di-$C_{1-4}$ alkylcarbamoylamino groups such as N,N-dimethylcarbamoylamino, N,N-diethylcarbamoylamino, N,N-dipropylcarbamoylamino and N,N-dibutylcarbamoylamino; alkylenedioxy groups having 1 to 3 carbon atoms such as methylenedioxy and ethylenedioxy; hydroxyl group; epoxy group (-O-); nitro group; cyano group; mercapto group; sulfo group; sulfino group; phosphono group; sulfamoyl group; monoalkylsulfamoyl groups having 1 to 6 carbon atoms such as N-methylsulfamoyl, N-ethylsulfamoyl, N-propylsulfamoyl, N-isopropylsulfamoyl, and N-butylsulfamoyl; di-$C_{1-4}$ alkylsulfamoyl groups such as N,N-dimethylsulfamoyl, N,N-diethylsulfamoyl, N,N-dipropylsulfamoyl and N,N-dibutylsulfamoyl; alkylthio groups having 1 to 6 carbon atoms such as methylthio, ethylthio, propylthio, isopropylthio, n-butylthio, sec-butylthio and tert-butylthio; phenylthio group; alkylsulfinyl groups having 1 to 6 carbon atoms such as methylsulfinyl, ethylsulfinyl, propylsulfinyl and butylsulfinyl; phenylsulfinyl group; alkylsulfonyl groups having 1 to 6 carbon atoms such as methylsulfonyl, ethylsulfonyl, propylsulfonyl and butylsulfonyl; and phenylsulfonyl group. The above-described groups may be

substituted with 1 to 5 of these substituents.

Examples of a heterocyclic group of the heterocyclic groups which may have a substituent represented by $R_1$ in the general formula (2) and $R_2$ in the general formula (3) include 5-membered cyclic groups containing, other than carbon atoms, 1 to 4 hetero atoms selected from oxygen atom, sulfur atom, nitrogen atom and the like, such as 2- or 3-thienyl, 2- or 3-furyl, 2- or 3-pyronyl, 2-, 3- or 4-pyridyl, 2-, 4- or 5-oxazolyl, 2-, 4- or 5-thiazolyl, 3, 4- or 5-pirazolyl, 2-, 4- or 5-imidazolyl, 3-, 4- or 5-isoxazolyl, 3-, 4- or 5-isothiazolyl, 3- or 5-(1,2,4-oxadiazolyl), 1,3,4-oxadiazolyl, 3- or 5-(1,2,4-thiadiazolyl), 1,3,4-thiadiazolyl, 4- or 5-(1,2,3-thiadiazolyl), 1,2,5-thiadiazolyl, 1,2,3-triazolyl, 1,2,4-triazolyl and 1H- or 2H-tetrazolyl; 6-membered cyclic groups containing, other than carbon atoms, 1 to 4 hetero atoms selected from oxygen atom, sulfur atom, nitrogen atom and the like, such as N-oxide-2-, 3- or 4-pyridyl, 2-, 4- or 5-pyrimidinyl, N-oxide-2-, 4- or 5-pyrimidinyl, thiomorpholinyl, morpholinyl, oxoimidazinyl, dioxotriazinyl, pyrolidinyl, piperidinyl, pyranyl, thiopyranyl, 1,4-oxazinyl, 1,4-thiazinyl, 1,3-thiazinyl, piperadinyl, triazinyl, oxotriazinyl, 3- or 4-pyridazinyl, pyrazinyl and N-oxide-3- or 4-pyridazinyl; 5 to 8-membered rings or the condensed rings containing 1 to 4 hetero atoms such as oxygen atom, sulfur atom, nitrogen atom and the like, in addition to carbon atoms in a 2 or 3 cyclic condensed ring group which contains, other than carbon atoms, 1 to 4 hetero atoms selected from oxygen atom, sulfur atom, nitrogen atom and the like, such as benzofuryl, benzothiazolyl, benzoxazolyl, tetrazolo[1,5-b]pyridazinyl, triazolo[4,5-b]pyridazinyl, benzoimidazolyl, quinolyl, isoquinolyl, cinnolinyl, phthalazinyl, quinazolinyl, quinoxalinyl, indolizinyl, quinolizinyl, 1,8-naphthylidinyl, prinyl, pteridinyl, dibenzofuranyl, carbazolyl, acridinyl, phenanthridinyl, chromanyl, benzoxazinyl, phenazinyl, phenothiazinyl, and phenoxazinyl.

Substituents of the heterocyclic groups are not specifically limited, and examples include those corresponding to the purposes and applications, such as hydrocarbon group and hetero atom-containing hydrocarbon groups. For example, substituents of the same kinds as those described above are properly included. The above-described groups may be substituted with 1 to 5 of these substituents.

Examples of an alkenyl group of the alkenyl groups which may have a substituent represented by $R_1$ in the general formula (2) and $R_2$ in the general formula (3) include alkenyl groups having 2 to 18 carbon atoms such as vinyl, allyl, methalyl, isopropenyl, 1-propenyl, 2-propenyl, 2-methyl-1-propenyl, butenyl, pentenyl, hexenyl, heptynyl, octenyl, nonenyl, decenyl, undecenyl, dodecenyl, tetradecenyl, hexadecenyl, and octadecenyl.

Substituents of the alkenyl groups are not specifically limited, and examples include those corresponding to the purposes and applications, such as hydrocarbon group and hetero atom-containing hydrocarbon groups. For example, substituents of the same kinds as those described above are included. The substituents may be substituted with 1 to 5 alkenyl groups.

Examples of a halogen atom represented by X in the general formula (2) include chlorine, bromine, and iodine.

Examples of an alkyl group of the alkyl group which may have a substituent represented by Ra in the general formula (3) include alkyl groups having 1 to 18 carbon atoms such as methyl, ethyl, propyl, iso-propyl, n-butyl, iso-butyl, sec-butyl, tert-butyl, pentyl, iso-pentyl, sec-pentyl, hexyl, heptyl, n-octyl, isooctyl, 2-ethylhexyl, nonyl, decyl, isodecyl, dodecyl, tetradecyl, hexadecyl, and octadecyl.

Substituents of the alkyl groups are not specifically limited, and examples include those corresponding to the purposes and applications, such as hydrocarbon group and hetero atom-containing hydrocarbon groups. For example, substituents of the same kinds as those described above are included. The substituents may be substituted with 1 to 5 alkyl groups.

Examples of an arylene group which may have a substituent, which substitutes for Ra and serves as a bond of-OBO- to form a ring containing -OBO- in the general formula (3), include arylene groups having 6 to 10 carbon atoms such as phenylene, tolylene, xylylene, and naphthylene.

Substituents of the arylene groups are not specifically limited, and examples include those corresponding to the purposes and applications, such as hydrocarbon group and hetero atom-containing hydrocarbon groups. For example, substituents of the same kinds as those described above are included. The substituents may be substituted with 1 to 5 arylene groups.

Examples of an alkylene group of the alkylene group which may have a substituent, which substitutes for Ra and serves as a bond of -OBO- to form a ring containing - OBO- in the general formula (3), include alkylene groups having 1 to 18 carbon atoms such as methylene, ethylene, propylene, iso-propylene, n-butylene, iso-butylene, sec-butylene, tert-butylene, pentylene, iso-pentylene, sec-pentylene, hexylene, heptylene, octylene, 2-ethylhexylene, nonylene, decylene, isodecylene, dodecylene, tetradecylene, hexadecylene, and octadecylene.

Substituents of the alkylene groups are not specifically limited, and examples include those corresponding to the purposes and applications, such as hydrocarbon group and hetero atom-containing hydrocarbon groups. For example, substituents of the same kinds as those described above are included. The substituents may be substituted with 1 to 5 alkylene groups.

When, in stead of Ra, a ring including -OBO- is formed through the above-described arylene group or alkylene serving as a bond of -OBO-, the above-described general formula (3) turns into the following general formula (7):

[Chemical Formula 1]

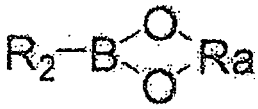

(7)

wherein $R_2$ represents an aryl group which may have a substituent, a heterocyclic group which may have a substituent or an alkenyl group which may have a substituent; and Ra represents an arylene group which may have a substituent or an alkylene group which may have a substituent.

More specifically, the general formula turns into the following general formula (8) when the arylene group which may have a substituent is phenylene, and it turns into the following general formula (9) when the alkylene group which may have a substituent is 1,1,2,2-tetramethylethylene.

[Chemical Formula 2]

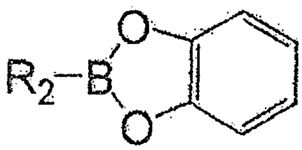

(8)

[Chemical Formula 3]

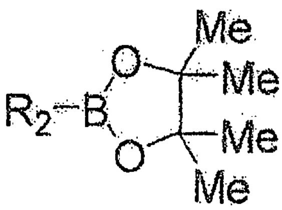

(9)

In the reaction of a compound represented by the above-described general formula (2) with a compound represented by the above-described general formula (3), a compound represented by the following general formula (10) is generated:

$$R_1\text{-}R_2 \qquad (10)$$

wherein $R_1$ and $R_2$ represent an aryl group which may have a substituent, a heterocyclic group which may have a substituent or an alkenyl group which may have a substituent.

In the general formula (10), an aryl group which may have a substituent, a heterocyclic group which may have a substituent or an alkenyl group which may have a substituent represented by $R_1$ and $R_2$ denote the same as described above.

In the reaction of a compound represented by the above-described general formula (1) with a compound represented by the above-described general formula (2), the method for synthesizing a compound of the present invention is given by the following reaction formula (11) called as Suzuki Cross-Couplings.

[Chemical Formula 4]

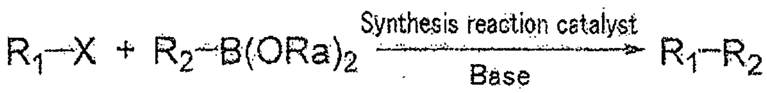


(11)

In the method for synthesizing a compound of the present invention, in the above-described reaction formula (11), a compound represented by the above-described general formula (1) and a compound represented by the above-described general formula (2) are reacted in the presence of the above-described layered perovskite-type composite oxide containing palladium and a base by using a reaction solvent.

In this reaction, examples of the base include inorganic salts such as hydroxides such as sodium hydroxide and potassium hydroxide; carbonates such as sodium carbonate ($Na_2CO_3$), potassium carbonate ($K_2CO_3$) and cesium carbonate ($Cs_2CO_3$); acetates such as sodium acetate and potassium acetate; phosphates such as sodium phosphate ($Na_3PO_4$) and potassium phosphate ($K_3PO_4$); and organic salts such as ammonium salts such as triethylamines, pyridine, morphorine, quinoline, piperidine, DBU (diazabicycloundecene), anilines, and tetra-n-butylammonium acetate. These bases can be used alone or in combination.

In this reaction, examples of the reaction solvent include water such as deionized water and distilled water; and aqueous solvents such as alcohols such as methanol, ethanol, 1-propanol, and isopropanol; and alkoxy alcohols such as 2-methoxy-1-propanol, 2-ethoxy-1-propanol, ethylene glycol monomethyl ether (methyl cellosolve), ethylene glycol monoethyl ether (ethyl cellosolve), and ethylene glycol isopropyl ether (isopropyl cellosolve). These reaction solvents can be used alone or in combination.

Of these, preferably, the reaction solvent containing glycol ether such as ethylene glycol monomethyl ether (methyl cellosolve), ethylene glycol monoethyl ether (ethyl cellosolve), and ethylene glycol isopropyl ether (isopropyl cellosolve), more preferably, a mixture solvent of glycol ether and other solvents, even more preferably, a mixture solvent of glycol ether / water, and particularly preferably, a mixture solvent of methyl cellosolve / deionized water is used.

In this reaction, a mixing ratio of a compound represented by the above-described general formula (1) to a compound represented by the above-described general formula (2) is not specifically limited. But, for example, the compound represented by the above-described general formula (2) is mixed in an amount of 0.1 to 10 equivalents, and preferably 0.5 to 2 equivalents, based on the compound represented by the above-described general formula (1).

Further, in this reaction, the layered perovskite-type composite oxide containing palladium is added, although it is not specifically limited, for example, in an amount of 0.01 to 0.00005 mol%, and preferably 0.001 to 0.0001 mol%, in terms of palladium content.

In this reaction, the base is added, although it is not specifically limited, for example, in an amount of 1 to 30 equivalents, and preferably 1 to 10 equivalents.

Also, in this reaction, the reaction solvent is not specifically limited, but is added in an amount of 100 to 3000 parts by weight, and preferably 300 to 1,000 parts by weight, based on 100 parts by weight of the mixing amount of the compound represented by the above-described general formula (1) and the compound represented by the above-described general formula (2).

In case of using a mixed solvent of a glycol ether and other solvents as the reaction solvent, a volume ratio of the glycol ether to the other solvent, glycol ether / the other solvent, is from 1 / 5 to 5 / 1, and preferably 1 /1.

The reaction is carried out, for example, under a reaction pressure of 0 to 5,000 KPa, and preferably from 0 to 3,000 KPa, at a reaction temperature of 0 to 250°C, and preferably from 0 to 150°C, for a reaction time of 0.1 to 72 hours, and preferably from 0.5 to 24 hours.

Further, in this reaction, an additive can be used in order to accelerate the reaction. Examples of the additive include organic ammonium halides such as tetra-n-butylammonium bromide (TBAB). The additive is added in an amount of, for example, 1 to 200 mol%.

More specifically, in this reaction, a compound represented by the above-described general formula (1) and a compound represented by the above-described general formula (2) are, together with the layered perovskite-type composite oxide containing palladium and a base, added to a reaction solvent in the above-described ratio, and reacted under the above-described reaction conditions, thereby to produce a compound represented by the above-described general formula (10).

In the method for synthesizing a compound of the present invention, a compound represented by the above-described general formula (10) can be synthesized in a high yield through Suzuki Cross-Couplings in the presence of the above-described layered perovskite-type composite oxide containing palladium.

Also, according to the method for synthesizing a compound of the present invention, in such a reaction, a catalyst for synthesis reaction of the present invention composed of the layered perovskite-type composite oxide containing palladium represented by the general formula of $Ln_2MyCu_{1-x-y}Pd_xO_{4\pm\delta}$ is used, so that by increasing the turnover number of the catalyst, even when a small amount of palladium is used, an activity of palladium can be effectively developed. Thus, the production cost of a catalyst can be reduced and as a result, a compound can be synthesized less expensively.

Therefore, the method for synthesizing a compound and a catalyst for synthesis reaction of the present invention can be effectively used in applications using Suzuki Cross-Couplings on the industrial basis, for example, synthesis of drugs having the following biphenyl skeletons.

[Chemical Formula 5]

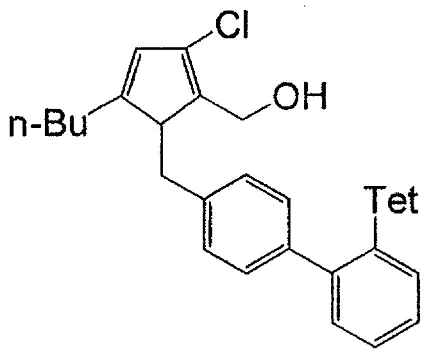

Tet :

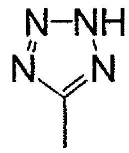

[Chemical Formula 6]

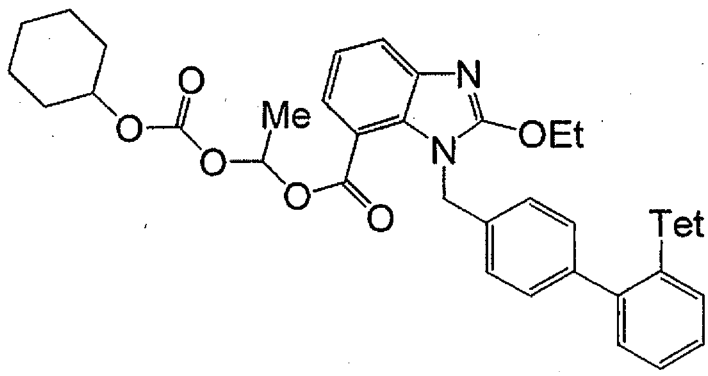

[Chemical Formula 7]

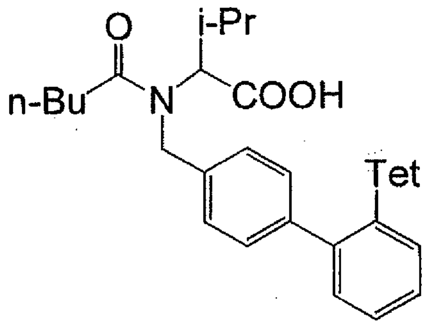

[Chemical Formula 8]

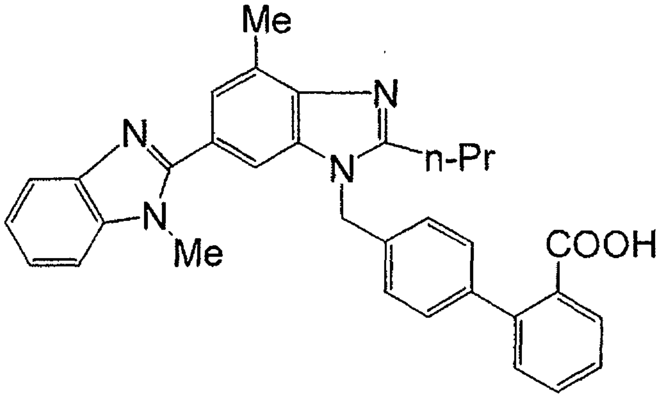

[Chemical Formula 9]

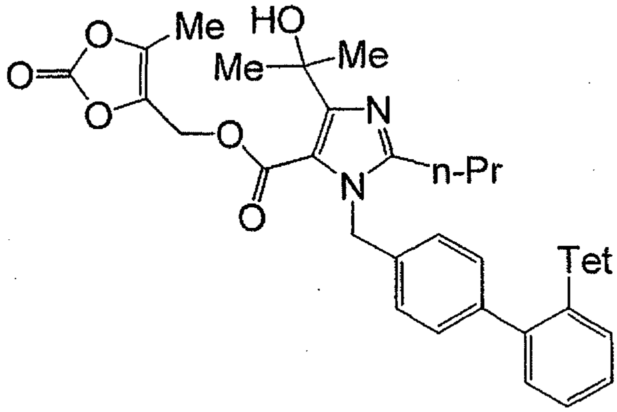

Examples

The present invention will now be described in more detail by way of Examples, but are not limited thereto.

1 Production Examples of Catalyst for Synthesis Reaction (layered perovskite-type composite oxide)

(1) Production Example 1 (Production of $La_2Cu_{0.95}Pd_{0.05}O_4$)

| | |
|---|---|
| Lanthanum ethoxyethylate | 8.12 g (0.020 mol) |
| Copper ethoxyethylate | 2.29 g (0.0095 mol) |

A mixed alkoxide solution was prepared by charging the above-described components in a 300 mL round-bottomed-flask, and dissolving them in 50 mL of toluene with stirring.

Next, 0.1525 g (0.00050 mol) of palladium acetylacetonate was dissolved in 20 mL of toluene, and was added to the mixed alkoxide solution to prepare a homogeneous mixed solution containing LaCuPd. Then, 50 mL of deionized water was added dropwise into the homogeneous mixed solution over about 15 minutes to be hydrolyzed. A viscous brown precipitate was formed on hydrolysis.

After stirring the viscous precipitate at room temperature for 2 hours, the toluene and the water were distilled off under reduced pressure conditions to produce a precursor of the LaCuPd composite oxide.

Then, the precursor was transferred into a petri dish, and subjected to forced-air drying at 60°C for 24 hours, subjected to a heat treatment (primary heat treatment) at 800°C in the atmosphere for 1 hour using an electric furnace to produce a blackish brown powder.

The X-ray powder diffraction of the powder was determined. The powder was identified as a crystal phase including a composite oxide having a layered perovskite-type structure of $La_2Cu_{0.95}Pd_{0.05}O_4$. The specific surface area

thereof was 4.5 $m^2/g$. The Pd content in the composite oxide was 1.31 mass%. The spectrum diagram of the X-ray diffraction is shown in FIG. 1.

(2) Production Example 2 (Production of $Pr_2Cu_{0.95}Pd_{0.05}O_4$)

| | |
|---|---|
| Praseodymium methoxypropylate | 8.16 g (0.020 mol) |
| Copper ethoxyethylate | 2.29 g (0.0095 mol) |

A mixed alkoxide solution was prepared by charging the above-described components in a 300 mL round-bottomed-flask, and dissolving them in 50 mL of toluene with stirring.

Next, 0.1525 g (0.00050 mol) of palladium acetylacetonate was dissolved in 20 mL of toluene, and was added to the mixed alkoxide solution to prepare a homogeneous mixed solution containing PrCuPd.

Hereinafter, in the same manner as in Production Example 1, a blackish brown powder was

The X-ray powder diffraction of the powder was determined. The powder was identified as a crystal phase including a composite oxide having a layered perovskite-type structure of $Pr_2Cu_{0.95}Pd_{0.05}O_4$. The specific surface area thereof was 2.0 $m^2/g$. The Pd content in the composite oxide was 1.29 mass%. The spectrum diagram of the X-ray diffraction is shown in FIG. 2.

(3) Production Example 3 (Production of $Nd_2Cu_{0.6}Pd_{0.4}O_4$)

| | |
|---|---|
| Neodymium n-butoxide | 7.27 g (0.020 mol) |
| Copper ethoxyethylate | 1.45 g (0.0060 mol) |

A mixed alkoxide solution was prepared by charging the above-described components in a 300 mL round-bottomed-flask, and dissolving them in 50 mL of toluene with stirring.

Next, 1.22 g (0.0040 mol) of palladium acetylacetonate was dissolved in 20 mL of toluene, and was added to the mixed alkoxide solution to prepare a homogeneous mixed solution containing NdCuPd.

Hereinafter, in the same manner as in Production Example 1, a blackish brown powder was obtained.

The X-ray powder diffraction of the powder was determined. The powder was identified as a crystal phase including a composite oxide having a layered perovskite-type structure of $Nd_2Cu_{0.6}Pd_{0.4}O_4$. The specific surface area thereof was 6.3 $m^2/g$. The Pd content in the composite oxide was 9.83 mass%. The spectrum diagram of the X-ray diffraction is shown in FIG. 3.

(4) Production Example 4 (Production of $Nd_2Cu_{0.8}Pd_{0.2}O_4$)

| | |
|---|---|
| Neodymium n-butoxide | 7.27 g (0.020 mol) |
| Copper ethoxyethylate | 1.93 g (0.0080 mol) |

A mixed alkoxide solution was prepared by charging the above-described components in a 300 mL round-bottomed-flask, and dissolving them in 50 mL of toluene with stirring.

Next, 0.61 g (0.0020 mol) of palladium acetylacetonate was dissolved in 20 mL of toluene, and was added to the mixed alkoxide solution to prepare a homogeneous mixed solution containing NdCuPd.

Hereinafter, in the same manner as in Production Example 1, a blackish brown powder was obtained.

The X-ray powder diffraction of the powder was determined. The powder was identified as a crystal phase including a composite oxide having a layered perovskite-type structure of -$Nd_2Cu_{0.8}Pd_{0.2}O_4$. The specific surface area thereof was 3.6 $m^2/g$. The Pd content in the composite oxide was 5.01 mass%. The spectrum diagram of the X-ray diffraction is shown in FIG. 4.

(5) Production Example 5 (Production of $Nd_2Cu_{0.95}Pd_{0.05}O_4$)

| | |
|---|---|
| Neodymium n-butoxide | 7.27 g (0.020 mol) |
| Copper ethoxyethylate | 2.29 g (0.0095 mol) |

A mixed alkoxide solution was prepared by charging the above-described components in a 300 mL round-bottomed-flask, and dissolving them in 50 mL of toluene with stirring.

Next, 0.1525 g (0.00050 mol) of palladium acetylacetonate was dissolved in 20 mL of toluene, and was added to the mixed alkoxide solution to prepare a homogeneous mixed solution containing NdCuPd.

Hereinafter, in the same manner as in Production Example 1, a blackish brown powder was obtained.

The X-ray powder diffraction of the powder was determined. The powder was identified as a crystal phase including a composite oxide having a layered perovskite-type structure of $Nd_2Cu_{0.95}Pd_{0.05}O_4$. The specific surface area thereof was 4.9 $m^2/g$. The Pd content in the composite oxide was 1.26 mass%. The spectrum diagram of the X-ray diffraction is shown in FIG. 5.

(6) Production Example 6 (Production of $Nd_2Cu_{0.99}Pd_{0.01}O_4$)

| | |
|---|---|
| Neodymium n-butoxide | 7.27 g (0.020 mol) |
| Copper ethoxyethylate | 2.39 g (0.0099 mol) |

A mixed alkoxide solution was prepared by charging the above-described components in a 300 mL round-bottomed-flask, and dissolving them in 50 mL of toluene with stirring.

Next, 0.0305 g (0.00010 mol) of palladium acetylacetonate was dissolved in 20 mL of toluene, and was added to the mixed alkoxide solution to prepare a homogeneous mixed solution containing NdCuPd.

Hereinafter, in the same manner as in Production Example 1 except that a precursor was subjected to a primary heat treatment at 600°C, a blackish brown powder was obtained.

The X-ray powder diffraction of the powder was determined. The powder was identified as a crystal phase including a composite oxide having a layered perovskite-type structure of $Nd_2Cu_{0.99}Pd_{0.01}O_4$. The specific surface area thereof was 12.0 $m^2/g$. The Pd content in the composite oxide was 0,26 mass%. The spectrum diagram of the X-ray diffraction is shown in FIG. 6.

(7) Production Example 7 (Production of $Nd_2Cu_{0.995}Pd_{0.0005}O_4$)

| | |
|---|---|
| Neodymium n-butoxide | 7.27 g (0.020 mol) |
| Copper ethoxyethylate | 2.40 g (0.00995 mol) |

A mixed alkoxide solution was prepared by charging the above-described components in a 300 mL round-bottomed-flask, and dissolving them in 50 mL of toluene with stirring.

Next, 0.01525 g (0.000050 mol) of palladium acetylacetonate was dissolved in 20 mL of toluene, and was added to the mixed alkoxide solution to prepare a homogeneous mixed solution containing NdCuPd.

Hereinafter, in the same manner as in Production Example 1, a blackish brown powder was obtained.

The X-ray powder diffraction of the powder was determined. The powder was identified as a crystal phase including a composite oxide having a layered perovskite-type structure of $Nd_2Cu_{0.995}Pd_{0.005}O_4$. The specific surface area thereof was 3.3 $m^2/g$. The Pd content in the composite oxide was 0.13 mass%. The spectrum diagram of the X-ray diffraction is shown in FIG. 7.

(8) Production Example 8 (Production of $Nd_2Cu_{0.999}Pd_{0.001}O_4$)

| | |
|---|---|
| Neodymium n-butoxide | 7.27 g (0.020 mol) |
| Copper ethoxyethylate | 2.41 g (0.00999 mol) |

A mixed alkoxide solution was prepared by charging the above-described components in a 300 mL round-bottomed-flask, and dissolving them in 50 mL of toluene with stirring.

Next, 0.00305 g (0.000010 mol) of palladium acetylacetonate was dissolved in 20 mL of toluene, and was added to the mixed alkoxide solution to prepare a homogeneous mixed solution containing NdCuPd.

Hereinafter, in the same manner as in Production Example 1, a blackish brown powder was obtained.

The X-ray powder diffraction of the powder was determined. The powder was identified as a crystal phase including a composite oxide having a layered perovskite-type structure of $Nd_2Cu_{0.999}Pd_{0.001}O_4$. The specific surface area thereof was 4.6 $m^2/g$. The Pd content in the composite oxide was 0.026 mass%. The spectrum diagram of the X-ray diffraction is shown in FIG. 8.

(9) Production Example 9 (Production of $Gd_2Cu_{0.95}Pd_{0.05}O_4$)

| | |
|---|---|
| Gadolinium n-butoxide | 7.53 g (0.020 mol) |
| Copper ethoxyethylate | 2.29 g (0.0095 mol) |

A mixed alkoxide solution was prepared by charging the above-described components in a 300 mL round-bottomed-flask, and dissolving them in 50 mL of toluene with stirring.

Next, 0.1525 g (0.00050 mol) of palladium acetylacetonate was dissolved in 20 mL of toluene, and was added to the mixed alkoxide solution to prepare a homogeneous mixed solution containing GdCuPd.

Hereinafter, in the same manner as in Production Example 1, a blackish brown powder was obtained.

The X-ray powder diffraction of the powder was determined. The powder was identified as a crystal phase including a composite oxide having a layered perovskite-type structure of $Gd_2Cu_{0.95}Pd_{0.05}O_4$. The specific surface area thereof was 2.9 $m^2/g$. The Pd content in the composite oxide was 1.20 mass%. The spectrum diagram of the X-ray diffraction is shown in FIG. 9.

(10) Production Example 10 (Production of $Gd_2Cu_{0.94}Pd_{0.01}O_{3.95}$)

| | |
|---|---|
| Gadolinium n-butoxide | 7.53 g (0.020 mol) |
| Copper ethoxyethylate | 2.27 g (0.0094 mol) |

A mixed alkoxide solution was prepared by charging the above-described components in a 300 mL round-bottomed-flask, and dissolving them in 50 mL of toluene with stirring.

Next, 0.0305 g (0.00010 mol) of palladium acetylacetonate was dissolved in 20 mL of toluene, and was added to the mixed alkoxide solution to prepare a homogeneous mixed solution containing GdCuPd.

Hereinafter, in the same manner as in Production Example 1, a blackish brown powder was obtained.

The X-ray powder diffraction of the powder was determined. The powder was identified as a crystal phase including a composite oxide having a layered perovskite-type structure of $Gd_2Cu_{0.94}Pd_{0.01}O_{3.95}$. The specific surface area thereof was 3.1 $m^2/g$. The Pd content in the composite oxide was 0.24 mass%. The spectrum diagram of the X-ray diffraction is shown in FIG. 10.

(11) Production Example 11 (Production of $Gd_2Cu_{0.995}Pd_{0.005}O_4$)

| | |
|---|---|
| Gadolinium n-butoxide | 7.53 g (0.020 mol) |
| Copper ethoxyethylate | 2.40 g (0.00995 mol) |

A mixed alkoxide solution was prepared by charging the above-described components in a 300 mL round-bottomed-flask, and dissolving them in 50 mL of toluene with stirring.

Next, 0.01525 g (0.000050 mol) of palladium acetylacetonate was dissolved in 20 mL of toluene, and was added to the mixed alkoxide solution to prepare a homogeneous mixed solution containing GdCuPd.

Hereinafter, in the same manner as in Production Example 1, a blackish brown powder was obtained.

The X-ray powder diffraction of the powder was determined. The powder was identified as a crystal phase including a composite oxide having a layered perovskite-type structure of $Gd_2Cu_{0.995}Pd_{0.005}O_{4.}$ The specific surface area thereof was 3.4 $m^2$/g. The Pd content in the composite oxide was 0.12 mass%. The spectrum diagram of the X-ray diffraction is shown in FIG. 11.

(12) Production Example 12 (Production of $(La_{0.6}Sr_{0.4})_2Cu_{0.95}Pd_{0.005}O_4$)

| | |
|---|---|
| Lanthanum ethoxyethylate | 4.87 g (0.012 mol) |
| Strontium methoxypropylate | 2.13 g (0.0080 mol) |
| Copper ethoxyethylate | 2.29 g (0.0095 mol) |

A mixed alkoxide solution was prepared by charging the above-described components in a 300 mL round-bottomed-flask, and dissolving them in 50 mL of toluene with stirring.

Next, 0.1525 g (0.00050 mol) of palladium acetylacetonate was dissolved in 20 mL of toluene, and was added to the mixed alkoxide solution to prepare a homogeneous mixed solution containing LaSrCuPd.

Hereinafter, in the same manner as in Production Example 1, a blackish brown powder was obtained.

The X-ray powder diffraction of the powder was determined. The powder was identified as a crystal phase including a composite oxide having a layered perovskite-type structure of $(La_{0.6}Sr_{0.4})_2Cu_{0.95}Pd_{0.05}O_4$. The specific surface area thereof was 3.7 $m^2$/g. The Pd content in the composite oxide was 1.45 mass%. The spectrum diagram of the X-ray diffraction is shown in FIG 12.

(13) Production Example 13 (Production of $(La_{0.06}Sr_{0.4})_2Cu_{0.94}Pd_{0.01}O_{3.95}$)

| | |
|---|---|
| Lanthanum ethoxyethylate | 4.87 g (0.012 mol) |
| Strontium methoxypropylate | 2.16 g (0.0080 mol) |
| Copper ethoxyethylate | 2.27 g (0.0094 mol) |

A mixed alkoxide solution was prepared by charging the above-described components in a 300 mL round-bottomed-flask, and dissolving them in 50 mL of toluene with stirring.

Next, 0.0305 g (0.00010 mol) of palladium acetylacetonate was dissolved in 20 mL of toluene, and was added to the mixed alkoxide solution to prepare a homogeneous mixed solution containing LaSrCuPd.

Hereinafter, in the same manner as in Production Example 1, a blackish brown powder was obtained.

The X-ray powder diffraction of the powder was determined. The powder was identified as a crystal phase including a composite oxide having a layered perovskite-type structure of $(La_{0.6}Sr_{0.4})_2Cu_{0.94}Pd_{0.01}O_{3.95}$. The specific surface area thereof was 2.7 $m^2$/g. The Pd content in the composite oxide was 0.29 mass%. The spectrum diagram of the X-ray diffraction is shown in FIG. 13.

(14) Production Example 14 (Production of $(Gd_{0.6}Sr_{0.4})_2Cu_{0.94}Pd_{0.01}O_{3.95}$)

| | |
|---|---|
| Gadolinium n-butoxide | 4.52 g (0.012 mol) |
| Strontium methoxypropylate | 2.13 g (0.0080 mol) |
| Copper ethoxyethylate | 2.27 g (0.0094 mol) |

A mixed alkoxide solution was prepared by charging the above-described components in a 300 mL round-bottomed-flask, and dissolving them in 50 mL of toluene with stirring.

Next, 0.0305 g (0.00010 mol) of palladium acetylacetonate was dissolved in 20 mL of toluene, and was added to the mixed alkoxide solution to prepare a homogeneous mixed solution containing GdSrCuPd.

Hereinafter, in the same manner as in Production Example 1, a blackish brown powder was obtained.

The X-ray powder diffraction of the powder was determined. The powder was identified as a crystal phase including a composite oxide having a layered perovskite-type structure of $(Gd_{0.6}Sr_{0.4})_2Cu_{0.94}Pd_{0.01}O_{3.95}$, The specific surface area thereof was 5.5 $m^2/g$. The Pd content in the composite oxide was 0.28 mass%. The spectrum diagram of the X-ray diffraction is shown in FIG. 14.

(15) Production Example 15 (Production of $(Nd_{0.92}Ce_{0.08})_2Cu_{0.95}Pd_{0.05}O_4$)

| | |
|---|---|
| Neodymium n-butoxide | 6.69 g (0.0184 mol) |
| Cerium methoxypropylate | 0.65 g (0.0016 mol) |
| Copper ethoxyethylate | 2.29 g (0.0095 mol) |

A mixed alkoxide solution was prepared by charging the above-described components in a 300 mL round-bottomed-flask, and dissolving them in 50 mL of toluene with stirring.

Next, 0.1525 g (0.00050 mol) of palladium acetylacetonate was dissolved in 20 mL of toluene, and was added to the mixed alkoxide solution to prepare a homogeneous mixed solution containing NdCeCuPd.

Hereinafter, in the same manner as in Production Example 1, a blackish brown powder was obtained.

The X-ray powder diffraction of the powder was determined. The powder was identified as a crystal phase including a composite oxide having a layered perovskite-type structure of $(Nd_{0.92}Ce_{0.08})_2Cu_{0.95}Pd_{0.05}O_4$. The specific surface area thereof was 6.0 $m^2/g$. The Pd content in the composite oxide was 1.27 mass%. The spectrum diagram of the X-ray diffraction is shown in FIG. 15.

(16) Production Example 16 (Production of $(La_{0.5}Y_{0.5})_2Cu_{0.95}Pd_{0.05}O_4$)

| | |
|---|---|
| Lanthanum ethoxyethylate | 4.06 g (0.010 mol) |
| Yttrium ethoxyethylate | 3.56 g (0.010 mol) |
| Copper ethoxyethylate | 2.29 g (0.0095 mol) |

A mixed alkoxide solution was prepared by charging the above-described components in a 300 mL round-bottomed-flask, and dissolving them in 50 mL of toluene with stirring.

Next, 0.1525 g (0.00050 mol) of palladium acetylacetonate was dissolved in 20 mL of toluene, and was added to the mixed alkoxide solution to prepare a homogeneous mixed solution containing LaYCuPd.

Hereinafter, in the same manner as in Production Example 1, a blackish brown powder was obtained.

The X-ray powder diffraction of the powder was determined. The powder was identified as a crystal phase including a composite oxide having a layered perovskite-type structure of $(La_{0.5}Y_{0.5})_2Cu_{0.95}Pd_{0.05}O_4$. The specific surface area thereof was 6.6 $m^2/g$. The Pd content in the composite oxide was 1.49 mass%. The spectrum diagram of the X-ray diffraction is shown in FIG. 16.

(17) Production Example 17 (Production of $LaFe_{0.95}Pd_{0.05}O_3$)

| | |
|---|---|
| Lanthanum isopropoxy ethylate | 4.48 g (0.010 mol) |
| Iron isopropoxy ethylate | 3.47 g (0.0095 mol) |

A mixed alkoxide solution was prepared by charging the above-described components in a 500 mL round-bottomed-flask, and dissolving them in 200 mL of toluene with stirring.

Next, 0.1525 g (0.00050 mol) of palladium acetylacetonate was dissolved in 100 mL of toluene, and was added to the mixed alkoxide solution to prepare a homogeneous mixed solution containing LaFePd. Then, 200 mL of deionized water was added dropwise into the homogeneous mixed solution over about 15 minutes to be hydrolyzed. A viscous

brown precipitate was formed on hydrolysis.

After stirring the viscous precipitate at room temperature for 2 hours, the toluene and the water were distilled off under reduced pressure conditions to produce a precursor of the LaFePd composite oxide.

Then, the precursor was transferred into a petri dish, and subjected to forced-air drying at 60°C for 24 hours, subjected to a heat treatment at 800°C in the atmosphere for 1 hour using an electric furnace to produce a blackish brown powder.

The X-ray powder diffraction of the powder was determined. The powder was identified as a crystal phase including a composite oxide having a perovskite-type structure of $LaFe_{0.95}Pd_{0.05}O_3$. The specific surface area thereof was 17.0 $m^2/g$. The Pd content in the composite oxide was 2.17 mass%. The spectrum diagram of the X-ray diffraction is shown in FIG. 17.

2 Measurement of Specific Surface Area

The respective powders obtained in Production Examples 1 to 17 were subjected to a secondary heat treatment under the conditions shown in Table 1, and the specific surface areas of the obtained respective powders were calculated by a BET method. The results are shown in Table 1 together with the specific surface areas and Pd contents of the powders obtained in Production Examples 1 to 17.

[Table 1]

Table 1

| | Composition of catalyst for synthesis reaction | Temperature/time of heat treatment | Specific surface area ($m^2/g$) | Pd content (wt%) |
|---|---|---|---|---|
| Production Example 1 | $La_2Cu_{0.95}Pd_{0.05}O_4$ | 800°C/1h | 4.5 | 1.31 |
| | | 1000°C/1h | 1.1 | |
| Production Example 2 | $Pr_2Cu_{0.95}Pd_{0.05}O_4$ | 800°/1h | 2.0 | 1.29 |
| | | 1000°C/1h | <0.1 | |
| Production Example 3 | $Nd_2Cu_{0.6}Pd_{0.4}O_4$ | 800°C/1h | 6.3 | 9.83 |
| | | 1000°C/1h | 0.8 | |
| Production Example 4 | $Nd_2Cu_{0.8}Pd_{0.2}O_4$ | 800°C/1h | 3.6 | 5.01 |
| | | 1000°C/1h | <0.1 | |
| Production Example 5 | $Nd_2Cu_{0.95}Pd_{0.05}O_4$ | 800°C/1h | 4.9 | 1.26 |
| | | 900°C/1h | 0.1 | |
| | | 1000°C/1h | <0.1 | |
| Production Example 6 | $Nd_2Cu_{0.99}Pd_{0.01}O_4$ | 600°C/1h | 12.0 | 0.26 |
| | | 700°C/1h | 5.9 | |
| | | 800°C/1h | 1.9 | |
| | | 900°C/1h | 0.1 | |
| | | 1000°C/1h | <0.1 | |
| | | 1100°C/1h | <0.1 | |
| Production Example 7 | $Nd_2CU_{0.995}Pd_{0.005}O_4$ | 800°C/1h | 3.3 | 0.13 |
| | | 1000°C/1h | <0.1 | |
| Production Example 8 | $Nd_2Cu_{0.999}Pd_{0.001}O_4$ | 800°C/1h | 4.6 | 0.026 |
| | | 1000°G/1h | <0.1 | |

(continued)

| | Composition of catalyst for synthesis reaction | Temperature/time of heat treatment | Specific surface area ($m^2/g$) | Pd content (wt%) |
|---|---|---|---|---|
| Production Example 9 | $Gd_2Cu_{0.95}P_{0.05}O_4$ | 800°C/1h | 2.9 | 1.20 |
| | | 900°C/1h | 0.1 | |
| | | 1000°C/1h | <0.1 | |
| Production Example 10 | $Gd_2Cu_{0.94}Pd_{0.01}O_{3.95}$ | 800°C/1h | 3.1 | 0.24 |
| | | 1000°C/1h | <0.1 | |
| Production Example 11 | $Gd_2Cu_{0.995}Pd_{0.005}O_4$ | 800°C/1h | 3.4 | 0.12 |
| | | 1000°C/1h | <0.1 | |
| Production Example 12 | $(La_{0.6}Sr_{0.4})_2Cu_{0.95}Pd_{0.05}O_4$ | 800°C/1h | 3.7 | 1.45 |
| | | 1000°C/1h | 1.7 | |
| Production Example 13 | $(La_{0.6}Sr_{0.4})_2Cu_{0.94}Pd_{0.01}O_{3.95}$ | 800°C/1h | 2.7 | 0.29 |
| | | 1000°C/1h | 1.8 | |
| Production Example 14 | $(Gd_{0..6}Sr_{0.4})_2Cu_{0.94}Pd_{0.01}O_{3.95}$ | 800°C/1h | 5.5 | 0.28 |
| | | 1000°C/1h | 0.7 | |
| Production Example 15 | $(Nd_{0.92}Ce_{0.08})_2Cu_{0.95}Pd_{0.05}O_4$ | 800°C/1h | 6.0 | 1.27 |
| | | 1000°C/1h | <0.1 | |
| Production Example 16 | $(La_{0.5}Y_{0.5})_2Cu_{0.95}Pd_{0.5}O_4$ | 800°C/1h | 6.0 | 1.49 |
| Production Example 17 | $LaFe_{0.95}Pd_{0.05}O_3$ | 800°C/1h | 17.0 | 2.17 |
| | | 1000°C/1h | 5.0 | |

3 Synthesis Example of 4-methoxybiphenyl by Suzuki Cross-Couplings (room temperature conditions)

In the presence of each of the layered perovskite-type composite oxides containing palladium (Pd catalyst) which were prepared in the above-described Production Examples and subjected to a secondary heat treatment as described above so as to have a specific surface area shown in Table 1, 4-bromoanisole and phenylboronic acid were reacted as shown in the following general formula (12).

[Chemical Formula 10]

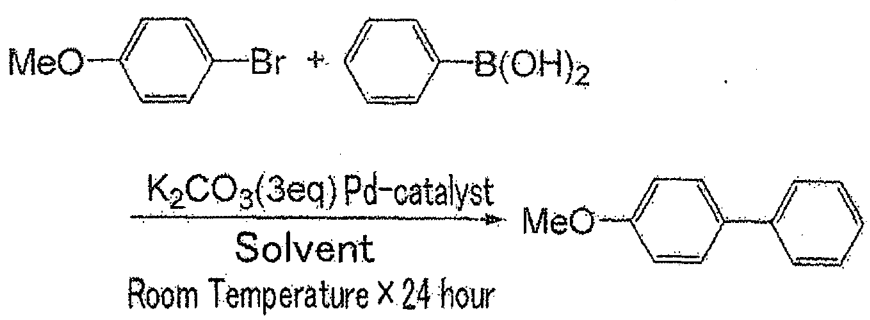


(12)

| | |
|---|---|
| 4-bromoanisole | 3.00 g (0.016 mol) |
| Phenylboronic acid | 2.93 g (0.024 mol) |

(continued)

| | |
|---|---|
| Potassium carbonate | 6.64 g (0.048 mol) |

The above-described components were charged in a 100 mL round-bottomed flask, and then 16 mL of methyl cellosolve (ethylene glycol monomethyl ether, hereinafter described as MC) and deionized water as a reaction solvent were each added thereto and cooled to room temperature. The layered perovskite-type composite oxide containing palladium prepared in each of the above-described Production Examples and after a secondary heat treatment was added to the solution in an amount shown in Table 2. Thereafter, the resulting solution was stirred at room temperature for 24 hours and the reaction was terminated. In Table 2, an amount of the layered perovskite-type composite oxide containing palladium was represented by mol% with respect to 4-bromoanisole. For example, 0.0002 mol% represents that $3.2 \times 10^{-8}$ mol of the layered perovskite-type composite oxide containing palladium was added as Pd.

After the reaction was terminated, 20 mL of toluene and 30 mL of deionized water were added to the reaction solution so as to dissolve the reaction product and salts which were produced as a by-product. Thereafter, an organic layer constituting the upper layer was analyzed with gas chromatography (GC) and the conversion rate was calculated according to the following equation. The results are shown in Table 2.

$$\text{Conversion rate (\%)} = \text{4-methoxybiphenyl} / (\text{4-bromoanisole} + \text{4-methoxybiphenyl}) \times 100$$

(As for 4-methoxybiphenyl and 4-bromoanisole, each toluene solution thereof was measured to determine the relative sensitivity and a calibration correction was performed in advance.)

Also, using gas chromatography as described above, the turnover number (TON) was calculated by the following equation in terms of mole number of the obtained 4-methoxybiphenyl per mol palladium. The results are shown in Table 2.

$$\text{Turnover number } (\text{Pd}^{-1}) = \text{4-methoxybiphenyl (mol)} / \text{palladium (mol)} \times \text{conversion rate}$$

[Table 2]

Table 2

| | Pd-containing perovskite-type composite oxide | | | | Reaction Conditions | | Conversion rate (%) | TON ($Pd^{-1}$) |
|---|---|---|---|---|---|---|---|---|
| | Composition of catalyst for synthesis reaction | Heat Treatment Temperature (°C) | Specific surface area ($m^2/g$) | Addition amount (Pd mol%) | Reaction Solvent | Reaction Temperature (°C) | | |
| Example 1 | $La_2Cu_{0.95}Pd_{0.05}O_4$ | 800 | 4.5 | 0.0002 | $MC/H_2O$ | Room Temperature | 40.3 | 110,500 |
| Example 2 | | 1000 | 1.1 | 0.0002 | $MC/H_2O$ | Room Temperature | 23.8 | 119,000 |
| Example 3 | $Pr_2Cu_{0.95}Pd_{0.05}O_4$ | 800 | 2.0 | 0.0002 | $MC/H_2O$ | Room Temperature | 33.0 | 165,000 |
| Example 4 | | 1000 | <0.1 | 0.0002 | $MC/H_2O$ | Room Temperature | 35.6 | 178,000 |
| Example 5 | $Nd_2Cu_{0.6}Pd_{0.4}O_4$ | 800 | 6.3 | 0.0002 | $MC/H_2O$ | Room Temperature | 33.1 | 165,500 |
| Example 6 | | 1000 | 0.8 | 0.0002 | $MC/H_2O$ | Room Temperature | 18.9 | 94,500 |
| Example 7 | $Nd_2Cu_{0.8}Pd_{0.2}O_4$ | 800 | 3.6 | 0.0002 | $MC/H_2O$ | Room Temperature | 41.7 | 208,500 |
| Example 8 | | 1000 | <0.1 | 0.0002 | $MC/H_2O$ | Room Temperature | 19.5 | 97,500 |
| Example 9 | $Nd_2Cu_{0.95}Pd_{0.5}O_4$ | 800 | 4.9 | 0.0002 | $MC/H_2O$ | Room Temperature | 27.7 | 138,500 |
| Example 10 | | 900 | 0.1 | 0.0002 | $MC/H_2O$ | Room Temperature | 38.9 | 194,500 |
| Example 11 | | 1000 | <0.1 | 0.0002 | $MC/H_2O$ | Room Temperature | 54.9 | 274,500 |

(continued)

| | Pd-containing perovskite-type composite oxide | | | | Reaction Conditions | | Conversion rate (%) | TON ($Pd^{-1}$) |
|---|---|---|---|---|---|---|---|---|
| | Composition of catalyst for synthesis reaction | Heat Treatment Temperature (°C) | Specific surface area ($m^2/g$) | Addition amount (Pd mol%) | Reaction Solvent | Reaction Temperature (°C) | | |
| Example 12 | $Nd_2Cu_{0.96}Pd_{0.01}O_4$ | 600 | 12.0 | 0.0002 | $MC/H_2O$ | Room Temperature | 20.3 | 101,500 |
| Example 13 | | 700 | 5.9 | 0.0002 | $MC/H_2O$ | Room Temperature | 21.9 | 109,500 |
| Example 14 | | 800 | 1.9 | 0.0002 | $MC/H_2O$ | Room Temperature | 48.8 | 244,000 |
| Example 15 | | 1000 | <0.1 | 0.0002 | $MC/H_2O$ | Room Temperature | 67.2 | 336,000 |
| Example 16 | $Nd_2Cu_{0.995}Pd_{0.005}O_4$ | 800 | 3.3 | 0.0002 | $MC/H_2O$ | Room Temperature | 99.4 | 497,000 |
| Example 17 | | 1000 | <0.1 | 0.0002 | $MC/H_2O$ | Room Temperature | 88.3 | 441,500 |
| Example 18 | $Nd_2Cu_{0.999}Pd_{0.001}O_4$ | 800 | 4.6 | 0.0002 | $MC/H_2O$ | Room Temperature | 52.0 | 260,000 |
| Example 19 | | 1000 | <0.1 | 0.0002 | $MC/H_2O$ | Room Temperature | 60.5 | 302,500 |
| Example 20 | $Gd_2Cu_{0.95}Pd_{0.05}O_4$ | 800 | 2.9 | 0.0002 | $MC/H_2O$ | Room Temperature | 59.6 | 298,000 |
| Example 21 | | 900 | 0.1 | 0.0002 | $MC/H_2O$ | Room Temperature | 15.7 | 78,500 |
| Example 22 | | 1000 | <0.1 | 0.0002 | $MC/H_2O$ | Room Temperature | 14.2 | 71,000 |
| Example 23 | $Gd_2Cu_{0.94}Pd_{0.01}O_{3.95}$ | 800 | 3.1 | 0.0002 | $MC/H_2O$ | Room Temperature | 84.9 | 424,500 |
| Example 24 | | 1000 | <0.1 | 0.0002 | $MC/H_2O$ | Room Temperature | 28.6 | 143,000 |

(continued)

| | Pd-containing perovskite-type composite oxide | | | | Reaction Conditions | | Conversion rate (%) | TON ($Pd^{-1}$) |
|---|---|---|---|---|---|---|---|---|
| | Composition of catalyst for synthesis reaction | Heat Treatment Temperature (°C) | Specific surface area ($m^2/g$) | Addition amount (Pd mol%) | Reaction Solvent | Reaction Temperature (°C) | | |
| Example 25 | $Gd_2Cu_{0.995}Pd_{0.05}O_4$ | 800 | 3.4 | 0.0002 | $MC/H_2O$ | Room Temperature | 88.3 | 441,500 |
| Example 26 | | 1000 | <0.1 | 0.0002 | $MC/H_2O$ | Room Temperature | 38.9 | 194,500 |
| Example 27 | $(La_{0.6}Sr_{0.4})_2Cu_{0.95}P_{0.05}O_4$ | 800 | 3.7 | 0.0002 | $MC/H_2O$ | Room Temperature | 34.3 | 171,500 |
| Example 28 | | 1000 | 1.7 | 0.0002 | $MC/H_2O$ | Room Temperature | 61.6 | 308,000 |
| Example 29 | $(La_{0.6}Sr_{0.4})_2Cu_{0.94}Pd_{0.01}O_{3.95}$ | 800 | 2.7 | 0.0002 | $MC/H_2O$ | Room Temperature | 68.5 | 342,500 |
| Example 30 | | 1000 | 1.8 | 0.0002 | $MC/H_2O$ | Room Temperature | 80.4 | 402,000 |
| Example 31 | $(Gd_{0.6}Sr_{0.4})_2Cu_{0.94}Pd_{0.01}O_{3.95}$ | 800 | 5.5 | 0.0002 | $MC/H_2O$ | Room Temperature | 45.5 | 227,500 |
| Example 32 | | 1000 | 0.7 | 0.0002 | $MC/H_2O$ | Room Temperature | 51.6 | 258,000 |
| Example 33 | $(Nd_{0.92}Ce_{0.09})_2Cu_{0.95}Pd_{0.05}O_4$ | 800 | 6.0 | 0.0002 | $MC/H_2O$ | Room Temperature | 22.2 | 111,000 |
| Example 34 | | 1000 | <0.1 | 0.0002 | $MC/H_2O$ | Room Temperature | 17.4 | 87,000 |
| Example 35 | $(La_{0.5}Y_{0.5})_2Cu_{0.95}Pd_{0.05}O_4$ | 800 | 6.0 | 0.0002 | $MC/H_2O$ | Room Temperature | 50.3 | 251,500 |
| Comparative Example 1 | $LaFe_{0.95}Pd_{0.05}O_3$ | 800 | 17.0 | 0.002 | $MC/H_2O$ | Room Temperature | 35.3 | 17,650 |
| Comparative Example 2 | | 1000 | 5.0 | 0.002 | $MC/H_2O$ | Room Temperature | 49.2 | 24,600 |

4 Synthesis Example of 4-methoxybiphenyl by Suzuki Cross-Couplings (80°C conditions)

In the presence of each of the layered perovskite-type composite oxides containing palladium (Pd catalyst) which were prepared in the above-described Production Examples and subjected to a secondary heat treatment as described above so as to have a specific surface area shown in Table 1, 4-bromoanisole and phenylboronic acid were reacted as shown in the following general formula (13).

[Chemical Formula 11]

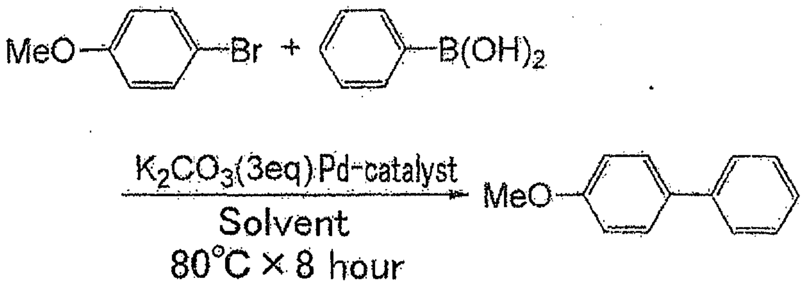


(13)

| | |
|---|---|
| 4-bromoanisole | 6.00 g (0.032 mol) |
| Phenylboronic acid | 5.86 g (0.048 mol) |
| Potassium carbonate | 13.28 g (0.096 mol) |

The above-described components were charged in a 200 mL round-bottomed flask, and then 32 mL of methyl cellosolve (ethylene glycol monomethyl ether, hereinafter described as MC) and deionized water as a reaction solvent were each added thereto and cooled to room temperature. The layered perovskite-type composite oxide containing palladium prepared in each of the above-described Production Examples and after a secondary heat treatment was added to the solution in an amount shown in Table 3. Thereafter, the resulting solution was gradually heated in a mantle heater, further heated till the temperature reached 80°C for about 1 hour, and stirred at 80°C for 8 hours, and then the reaction was terminated. In Table 3, an amount of the layered perovskite-type composite oxide containing palladium was represented by mol% with respect to 4-bromoanisole. For example, 0.0001 mol% represents that $3.2 \times 10^{-8}$ mol of the layered perovskite-type composite oxide containing palladium was added as Pd.

After the reaction was terminated, the reaction solution was cooled, and 40 mL of toluene and 60 mL of deionized water were added thereto so as to dissolve the reaction product and salts which were produced as a by-product. Thereafter, an organic layer constituting the upper layer was analyzed with gas chromatography (GC) and the conversion rate was calculated according to the following equation. The results are shown in Table 3.

$$\text{Conversion rate (\%)} = \text{4-methoxybiphenyl} / (\text{4-bromoanisole} + \text{4-methoxybiphenyl}) \times 100$$

(As for 4-methoxybiphenyl and 4-bromoanisole, each toluene solution thereof was measured to determine the relative sensitivity and a calibration correction was performed in advance.)

Also, using gas chromatography as described above, the turnover number (TON) was calculated by the following equation in terms of mole number of the obtained 4-methoxybiphenyl per mol palladium. The results are shown in Table 3.

$$\text{Turnover number } (Pd^{-1}) = \text{4-methoxybiphenyl (mol)} / \text{palladium (mol)} \times \text{conversion rate}$$

[Table 3]

Table 3

| | Pd-containing perovskite-type composite oxide | | | | Reaction Conditions | | Conversion rate (%) | TON ($Pd^{-1}$) |
|---|---|---|---|---|---|---|---|---|
| | Composition of catalyst for synthesis reaction | Heat Treatment Temperature (°C) | Specific surface area ($m^2/g$) | Addition amount (Pd mol%) | Reaction Solvent | Reaction Temperature (°C) | | |
| Example 36 | $La_2Cu_{0.95}Pd_{0.05}O_4$ | 1000 | 1.1 | 0.0001 | $MC/H_2O$ | 80 | 93.7 | 937,000 |
| Example 37 | $Nd_2Cu_{0.6}Pd_{0.4}O_4$ | 800 | 6.3 | 0.0001 | $MC/H_2O$ | 80 | 74.4 | 744,000 |
| Example 38 | | 1000 | <0.1 | 0.0001 | $MC/H_2O$ | 80 | 95.4 | 954,000 |
| Example 39 | $Nd_2Cu_{0.99}Pd_{0.01}O_4$ | 600 | 12.0 | 0.0001 | $MC/H_2O$ | 80 | 99.7 | 997,000 |
| Example 40 | | 700 | 5.9 | 0.0001 | $MC/H_2O$ | 80 | 84.2 | 842,000 |
| Example 41 | | 800 | 1.9 | 0.0001 | $MC/H_2O$ | 80 | 98.5 | 985,000 |
| Example 42 | | 900 | 0.1 | 0.0001 | $MC/H_2O$ | 80 | 83.4 | 834,000 |
| Example 43 | | 1000 | <0.1 | 0.0001 | $MC/H_2O$ | 80 | 95.7 | 957,000 |
| Example 44 | | 1100 | <0.1 | 0.0001 | $MC/H_2O$ | 80 | 98.2 | 982,000 |
| Example 45 | $Nd_2Cu_{0.995}M_{0.005}O_4$ | 800 | 3.3 | 0.0001 | $MC/H_2O$ | 80 | 97.0 | 970,000 |
| Example 46 | | 1000 | <0.1 | 0.0001 | $MC/H_2O$ | 80 | 99.0 | 990,000 |
| Example 47 | $Gd_2Cu_{0.995}Pd_{0.005}O_4$ | 800 | 3.4 | 0.0001 | $MC/H_2O$ | 80 | 93.4 | 934,000 |
| Example 48 | | 1000 | <0.1 | 0.0001 | $MC/H_2O$ | 80 | 96.8 | 968,000 |
| Example 49 | $(La_{0.6}Sr_{0.4})_2CU_{0.94}Pd_{0.01}O_{3.95}$ | 800 | 2.7 | 0.0001 | $MC/H_2O$ | 80 | 96.7 | 967,000 |
| Example 50 | | 1000 | 1.8 | 0.0001 | $MC/H_2O$ | 80 | 92.7 | 927,000 |
| Comparative Example 3 | $LaFe_{0.95}Pd_{0.05}O_3$ | 800 | 17.0 | 0.0001 | $MC/H_2O$ | 80 | 48.0 | 480,000 |
| Comparative Example 4 | | 1000 | 5.0 | 0.0001 | $MC/H_2O$ | 80 | 56.0 | 560,000 |

While the illustrative embodiments and examples of the present invention are provided in the above description, such is for illustrative purpose only and it is not to be construed limitative.

Industrial Applicability

The method for synthesizing a compound of the present invention and the catalyst for synthesis reaction of the present invention are effectively applied in Suzuki Cross-Couplings.

## Claims

1. A method for synthesizing a compound, which comprises reacting a compound represented by the following general formula (2) with a compound represented by the following general formula (3) in the presence of a palladium-containing layered perovskite-type composite oxide represented by the following general formula (1):

$$Ln_2M_yCu_{1-x-y}Pd_xO_{4\pm\delta} \quad (1)$$

wherein Ln represents elements comprising at least one essential element selected from La, Pr, Nd, Sm, Eu, and Gd and at least one optional element selected from Y, Ce, Yb, Ca, Sr, and Ba; M represents at least one element selected from Cr, Mn, Fe, Co, Ni, and A1; x, indicating an atomic proportion, is $0.001 \leq x\ 0.4$; y, indicating an atomic proportion, is $0 \leq y \leq 0.5$; and $\delta$ indicates an oxygen excess amount or an oxygen deficiency amount,

$$R_1\text{-}X \quad (2)$$

wherein $R_1$ represents an aryl group which may have a substituent, a heterocyclic group which may have a substituent, or an alkenyl group which may have a substituent; and X represents a halogen atom, a trifluoromethanesulfonyloxy group, a p-toluenesulfonyloxy group or a methanesulfonyloxy group, and

$$R_2\text{-}B(ORa)_2 \quad (3)$$

wherein $R_2$ represents an aryl group which may have a substituent, a heterocyclic group which may have a substituent, or an alkenyl group which may have a substituent; and Ra represents a hydrogen atom or an alkyl group which may have a substituent. Instead of Ra, a ring including -OBO- may be formed through an arylene group which may have a substituent or an alkylene group which may have a substituent, both of which serve as a bond of -OBO-.

2. The method for synthesizing a compound according to claim 1, wherein in the above-described general formula (1), Ln is at least one element selected from La, Nd, and Gd.

3. The method for synthesizing a compound according to claim 1, wherein in the above-described general formula, x represents an atomic proportion satisfying the following relation: $0.005 \leq x \leq 0.05$.

4. The method for synthesizing a compound according to claim 1, wherein a compound represented by the above-described general formula (2) is reacted with a compound represented by the above-described general formula (3) by using a reaction solvent containing a glycol ether.

5. A catalyst for synthesis reaction comprising a palladium-containing layered perovskite-type composite oxide which is represented by the following general formula (1), and is used so as to react a compound represented by the following general formula (2) with a compound represented by the following general formula (3):

$$Ln_2M_yCu_{1-x-y}Pd_xO_{4\pm5} \quad (1)$$

wherein Ln represents elements comprising at least one essential element selected from La, Pr, Nd, Sm, Eu, and Gd and at least one optional element selected from Y, Ce, Yb, Ca, Sr, and Ba; M represents at least one element selected from Cr, Mn, Fe, Co, Ni, and A1; x, indicating an atomic proportion, is $0.001 \leq x \leq 0.4$; y, indicating an atomic proportion, is $0 \leq y \leq 0.5$; and $\delta$ indicates an oxygen excess amount or an oxygen deficiency amount,

$$R_1\text{-}X \quad (2)$$

wherein $R_1$ represents an aryl group which may have a substituent, a heterocyclic group which may have a substituent, or an alkenyl group which may have a substituent; and X represents a halogen atom, a trifluoromethanesulfonyloxy group, a p-toluenesulfonyloxy group or a methanesulfonyloxy group, and

$$R_2\text{-B(ORa)}_2 \qquad (3)$$

wherein $R_2$ represents an aryl group which may have a substituent, a heterocyclic group which may have a substituent, or an alkenyl group which may have a substituent; and Ra represents a hydrogen atom or an alkyl group which may have a substituent. Instead of Ra, a ring including -OBO- may be formed through an arylene group which may have a substituent or an alkylene group which may have a substituent, both of which serve as a bond of -OBO-.

6. The catalyst for synthesis reaction according to claim 5, wherein in the above-described general formula (1), Ln is at least one element selected from La, Nd, and Gd.

7. The catalyst for synthesis reaction according to claim 5, wherein in the above-described general formula, x represents an atomic proportion satisfying the following relation: $0.005 \leq x \leq 0.05$.

**Patentansprüche**

1. Verfahren zur Synthese einer Verbindung, welches Umsetzen einer Verbindung, die durch die folgende allgemeine Formel (2) dargestellt wird, mit einer Verbindung, die durch die folgende allgemeine Formel (3) dargestellt wird, in der Gegenwart eines palladiumhaltigen Kompositoxids vom Schichtperovskit-Typ, das durch die folgende allgemeine Formel (1) dargestellt wird, umfasst:

$$Ln_2M_yCu_{1-x-y}Pd_xO_{4\pm\delta} \qquad (1)$$

wobei Ln Elemente darstellt, die mindestens ein wesentliches Element, ausgewählt aus La, Pr, Nd, Sm, Eu und Gd, und mindestens ein optionales Element, ausgewählt aus Y, Ce, Yb, Ca, Sr und Ba, umfassen; M mindestens ein Element darstellt, das aus Cr, Mn, Fe, Co, Ni und Al ausgewählt ist; für x, welches für einen Atomanteil steht, $0{,}001 \leq x \leq 0{,}4$ gilt; für y, welches für einen Atomanteil steht, $0 \leq y \leq 0{,}5$ gilt; und $\delta$ für eine Sauerstoffüberschussmenge oder eine Sauerstoffunterschussmenge steht,

$$R_1\text{-X} \qquad (2)$$

wobei $R_1$ einen Arylrest, der einen Substituenten aufweisen kann, einen heterocyclischen Rest, der einen Substituenten aufweisen kann, oder einen Alkenylrest, der einen Substituenten aufweisen kann, darstellt; und X ein Halogenatom, eine Trifluormethansulfonyloxygruppe, eine p-Toluolsulfonyloxy-gruppe oder eine Methansulfonyloxygruppe darstellt, und

$$R_2\text{-B(ORa)}_2 \qquad (3)$$

wobei $R_2$ einen Arylrest, der einen Substituenten aufweisen kann, einen heterocyclischen Rest, der einen Substituenten aufweisen kann, oder einen Alkenylrest, der einen Substituenten aufweisen kann, darstellt; und Ra ein Wasserstoffatom oder einen Alkylrest, der einen Substituenten aufweisen kann, darstellt, wobei anstelle von Ra ein Ring, einschließlich -OBO-, durch einen Arylenrest, der einen Substituenten aufweisen kann, oder einen Alkylenrest, der einen Substituenten aufweisen kann, welche beide als eine Bindung von -OBO- dienen, gebildet werden kann.

2. Verfahren zur Synthese einer Verbindung gemäß Anspruch 1, wobei in der vorstehend beschriebenen allgemeinen Formel (1) Ln mindestens ein Element, ausgewählt aus La, Nd und Gd, ist.

3. Verfahren zur Synthese einer Verbindung gemäß Anspruch 1, wobei in der vorstehend beschriebenen allgemeinen Formel x einen Atomanteil darstellt, der die folgende Relation erfüllt: $0{,}005 \leq x \leq 0{,}05$.

4. Verfahren zur Synthese einer Verbindung gemäß Anspruch 1, wobei eine Verbindung, die durch die vorstehend beschriebene allgemeine Formel (2) dargestellt wird, mit einer Verbindung, die durch die vorstehend beschriebene allgemeine Formel (3) dargestellt wird, unter Verwendung eines Reaktionslösungsmittels, welches einen Glycolether enthält, umgesetzt wird.

5. Katalysator für Synthesereaktion, umfassend ein palladiumhaltiges Kompositoxid vom Schichtperovskit-Typ, welches durch die folgende allgemeine Formel (1) dargestellt wird und so zur Umsetzung einer Verbindung, welche durch die folgende allgemeine Formel (2) dargestellt wird, mit einer Verbindung, welche durch die folgende allgemeine Formel (3) dargestellt wird, verwendet wird:

$$Ln_2M_yCu_{1\text{-}x\text{-}y}Pd_x0_{4\pm\delta} \quad (1)$$

wobei Ln Elemente darstellt, die mindestens ein wesentliches Element, ausgewählt aus La, Pr, Nd, Sm, Eu und Gd, und mindestens ein optionales Element, ausgewählt aus Y, Ce, Yb, Ca, Sr und Ba, umfassen; M mindestens ein Element darstellt, das aus Cr, Mn, Fe, Co, Ni und A1 ausgewählt ist; für x, welches für einen Atomanteil steht, $0{,}001 \leq x \leq 0{,}4$ gilt; für y, welches für einen Atomanteil steht, $0 \leq y \leq 0{,}5$ gilt; und $\delta$ für eine Sauerstoffüberschussmenge oder eine Sauerstoffunterschussmenge steht,

$$R_1\text{-}X \quad (2)$$

wobei $R_1$ einen Arylrest, der einen Substituenten aufweisen kann, einen heterocyclischen Rest, der einen Substituenten aufweisen kann, oder einen Alkenylrest, der einen Substituenten aufweisen kann, darstellt; und X ein Halogenatom, eine Trifluormethansulfonyloxygruppe, eine p-Toluolsulfonyloxy-gruppe oder eine Methansulfonyloxygruppe darstellt, und

$$R_2\text{-}B(ORa)_2 \quad (3)$$

wobei $R_2$ einen Arylrest, der einen Substituenten aufweisen kann, einen heterocyclischen Rest, der einen Substituenten aufweisen kann, oder einen Alkenylrest, der einen Substituenten aufweisen kann, darstellt; und Ra ein Wasserstoffatom oder einen Alkylrest, welcher einen Substituenten aufweisen kann, darstellt, wobei anstelle von Ra ein Ring, einschließlich -OBO-, durch einen Arylenrest, der einen Substituenten aufweisen kann, oder einen Alkylenrest, der einen Substituenten aufweisen kann, welche beide als eine Bindung von -OBO- dienen, gebildet werden kann.

6. Katalysator für Synthesereaktion gemäß Anspruch 5, wobei in der vorstehend beschriebenen allgemeinen Formel (1) Ln mindestens ein Element, ausgewählt aus La, Nd und Gd, ist.

7. Katalysator für Synthesereaktion gemäß Anspruch 5, wobei in der vorstehend beschriebenen allgemeinen Formel x einen Atomanteil darstellt, der die folgende Relation erfüllt: $0{,}005 \leq x \leq 0{,}05$.

## Revendications

1. Procédé pour synthétiser un composé, qui comprend la réaction d'un composé représenté par la formule générale (2) suivante avec un composé représenté par la formule générale (3) suivante en présence d'un oxyde composite de type perovskite lamellaire contenant du palladium représenté par la formule générale (1) suivante :

$$Ln_2M_yCu_{1\text{-}x\text{-}y}Pd_xO_{4\pm\delta} \quad (1)$$

dans laquelle Ln représente des éléments comprenant au moins un élément essentiel choisi parmi La, Pr, Nd, Sm, Eu et Gd et au moins un élément optionnel choisi parmi Y, Ce, Yb, Ca, Sr et Ba ; M représente au moins un élément choisi parmi Cr, Mn, Fe, Co, Ni et Al ; x indique une proportion atomique et $0{,}001 \leq x \leq 0{,}4$ ; y indique une proportion atomique et $0 \leq y \leq 0{,}5$ ; et $\delta$ indique une quantité d'oxygène en excès ou une quantité d'oxygène en défaut,

$$R_1\text{-}X \quad (2)$$

dans laquelle $R_1$ représente un groupe aryle pouvant porter un substituant, un groupe hétérocyclique pouvant porter un substituant, ou un groupe alcényle pouvant porter un substituant ; et X représente un atome d'halogène, un groupe trifluorométhanesulfonyloxy, un groupe p-toluènesulfonyloxy ou un groupe méthanesulfonyloxy, et

$$R_2\text{-}B(ORa)_2 \quad (3)$$

dans laquelle $R_2$ représente un groupe aryle pouvant porter un substituant, un groupe hétérocyclique pouvant porter

un substituant, ou un groupe alcényle pouvant porter un substituant ; et Ra représente un atome d'hydrogène ou un groupe alkyle pouvant porter un substituant. A la place de Ra, un cycle contenant -OBO- peut être formé par l'intermédiaire d'un groupe arylène pouvant porter un substituant ou d'un groupe alkylène pouvant porter un substituant, les deux servant de liaison de -OBO-.

**2.** Procédé pour synthétiser un composé selon la revendication 1, dans lequel, dans la formule générale (1) décrite ci-dessus, Ln est au moins un élément choisi parmi La, Nd et Gd.

**3.** Procédé pour synthétiser un composé selon la revendication 1, dans lequel, dans la formule générale décrite ci-dessus, x représente une proportion atomique satisfaisant à la relation suivante : $0{,}005 \leq x \leq 0{,}05$.

**4.** Procédé pour synthétiser un composé selon la revendication 1, dans lequel un composé représenté par la formule générale (2) décrite ci-dessus est mis à réagir avec un composé représenté par la formule générale (3) décrite ci-dessus en utilisant un solvant réactionnel contenant un éther de glycol.

**5.** Catalyseur pour réaction de synthèse comprenant un oxyde composite de type perovskite lamellaire contenant du palladium qui est représenté par la formule générale (1) suivante, et est utilisé de façon à faire réagir un composé représenté par la formule générale (2) suivante avec un composé représenté par la formule générale (3) suivante :

$$Ln_2M_yCu_{1-x-y}Pd_xO_{4\pm\delta} \qquad (1)$$

dans laquelle Ln représente des éléments comprenant au moins un élément essentiel choisi parmi La, Pr, Nd, Sm, Eu et Gd et au moins un élément optionnel- choisi parmi Y, Ce, Yb, Ca, Sr et Ba ; M représente au moins un élément choisi parmi Cr, Mn, Fe, Co, Ni et Al ; x indique une proportion atomique et $0{,}001 \leq x \leq 0{,}4$ ; y indique une proportion atomique et $0 \leq y \leq 0{,}5$ ; et $\delta$ indique une quantité d'oxygène en excès ou une quantité d'oxygène en défaut,

$$R_1\text{-}X \qquad (2)$$

dans laquelle $R_1$ représente un groupe aryle pouvant porter un substituant, un groupe hétérocyclique pouvant porter un substituant, ou un groupe alcényle pouvant porter un substituant ; et X représente un atome d'halogène, un groupe trifluorométhanesulfonyloxy, un groupe p-toluènesulfonyloxy ou un groupe méthanesulfonyloxy, et

$$R_2\text{-}B\ (ORa)_2 \qquad (3)$$

dans laquelle $R_2$ représente un groupe aryle pouvant porter un substituant, un groupe hétérocyclique pouvant porter un substituant, ou un groupe alcényle pouvant porter un substituant ; et Ra représente un atome d'hydrogène ou un groupe alkyle pouvant porter un substituant. A la place de Ra, un cycle contenant -OBO- peut être formé par l'intermédiaire d'un groupe arylène pouvant porter un substituant ou d'un groupe alkylène pouvant porter un substituant, les deux servant de liaison de -OBO-.

**6.** Catalyseur pour réaction de synthèse selon la revendication 5, dans lequel, dans la formule générale (1) décrite ci-dessus, Ln est au moins un élément choisi parmi La, Nd et Gd.

**7.** Catalyseur pour réaction de synthèse selon la revendication 5, dans lequel, dans la formule générale décrite ci-dessus, x représente une proportion atomique satisfaisant à la relation suivante : $0{,}005 \leq x \leq 0{,}05$.

FIG. 1

$La_2Cu_{0.95}Pd_{0.05}O_4$

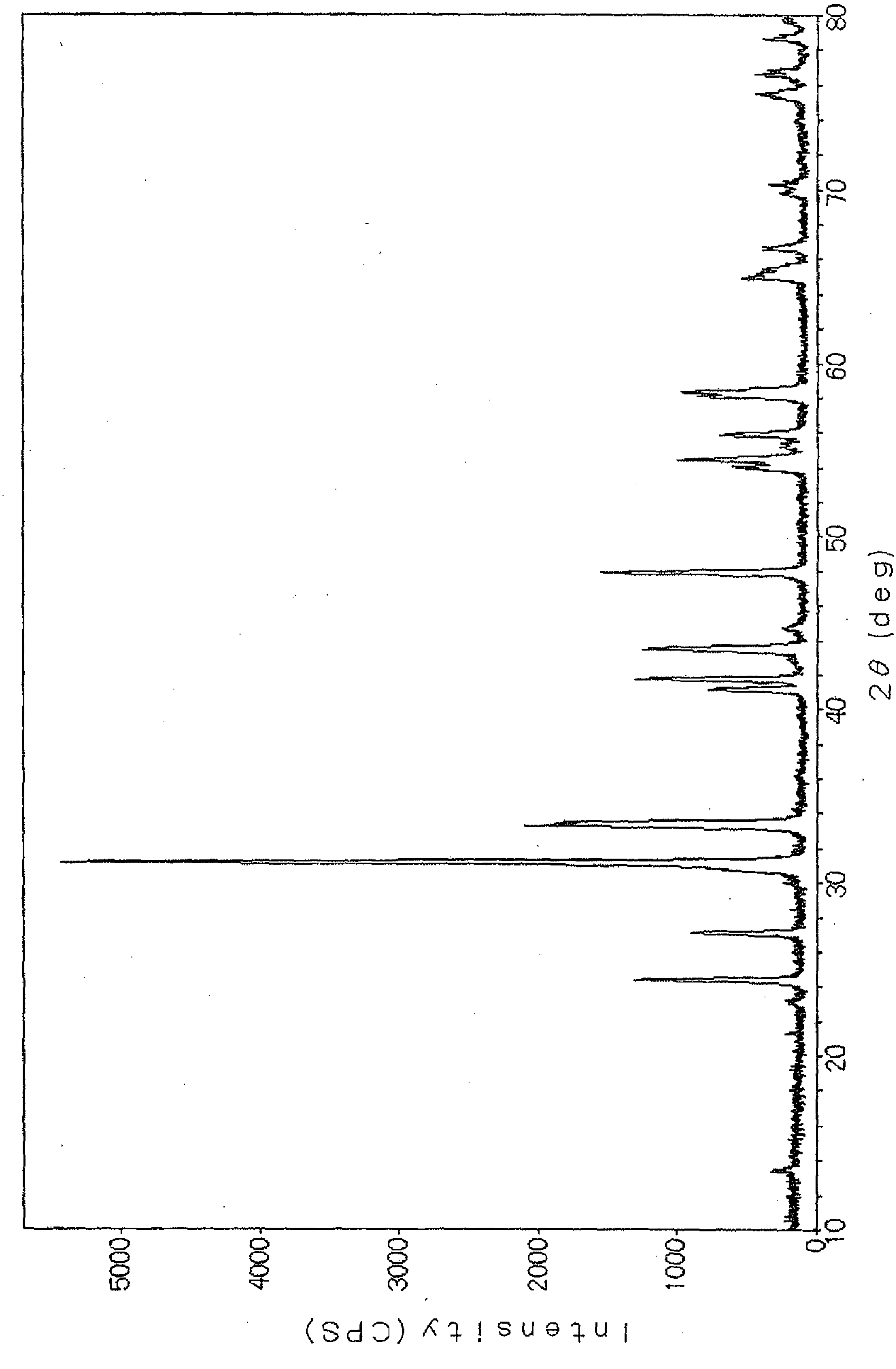

FIG. 2 $Pr_2Cu_{0.95}Pd_{0.05}O_4$

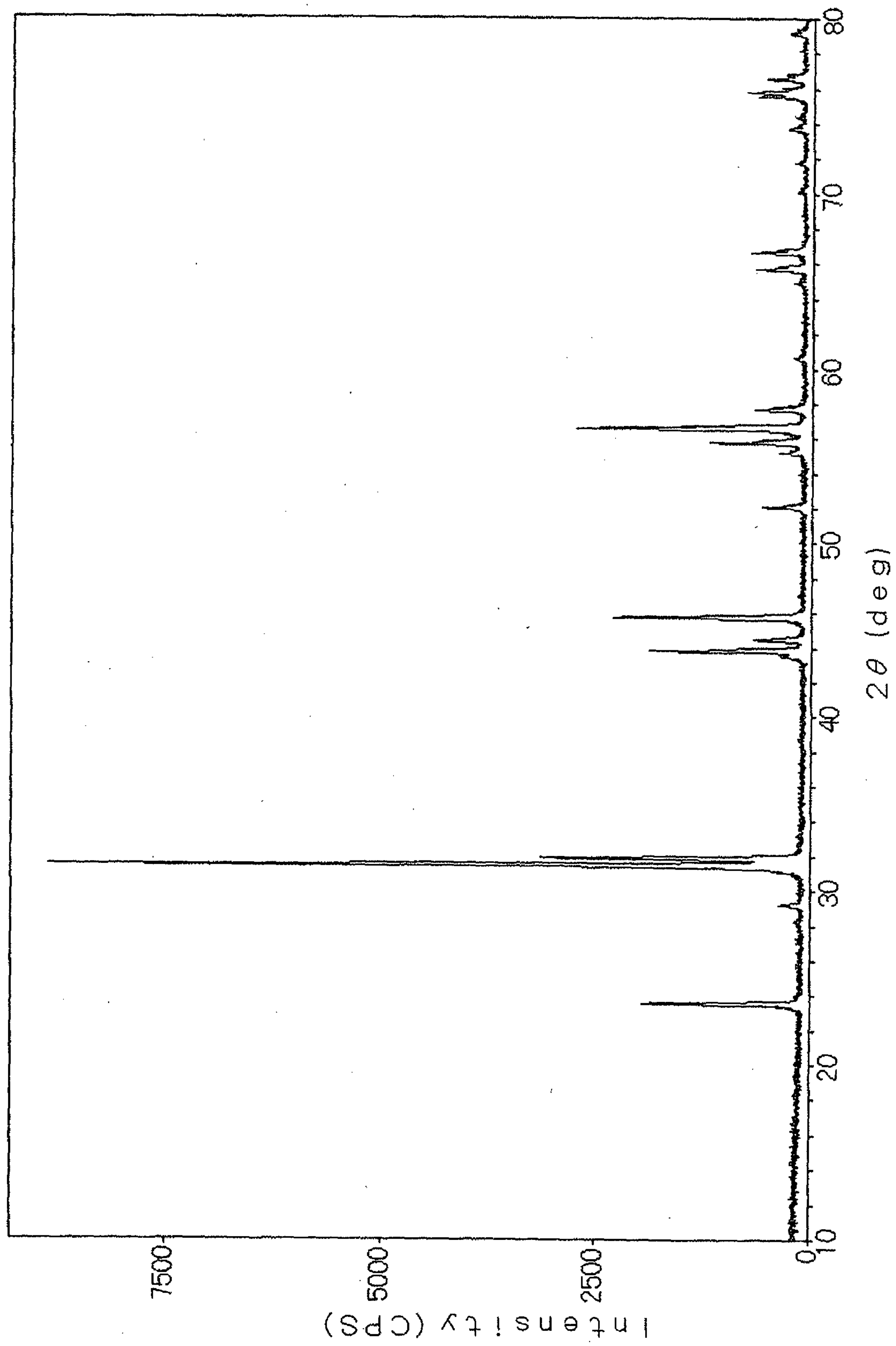

FIG. 3

$Nd_2Cu_{0.6}Pd_{0.4}O_4$

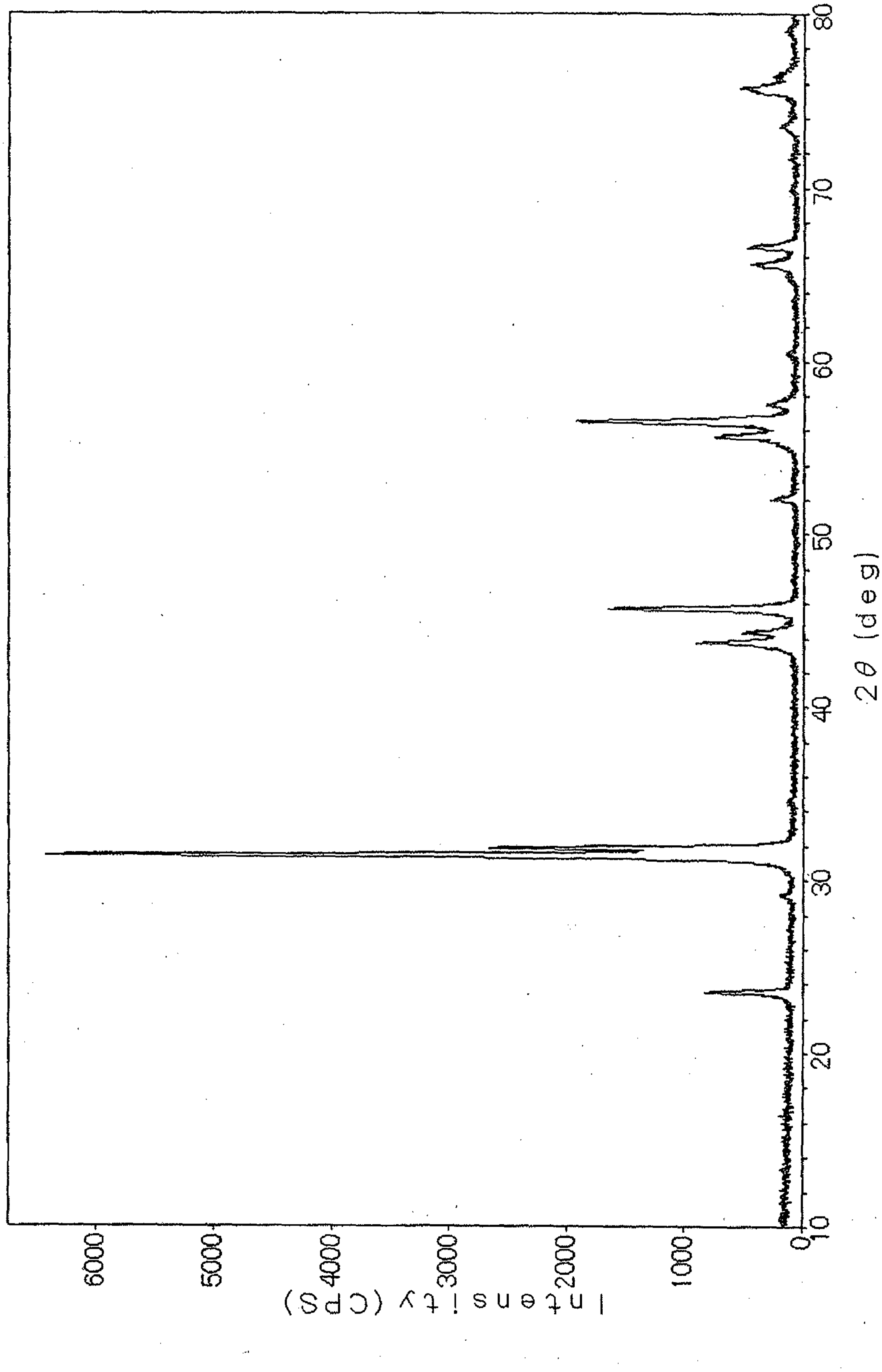

FIG. 4 $Nd_2Cu_{0.8}Pd_{0.2}O_4$

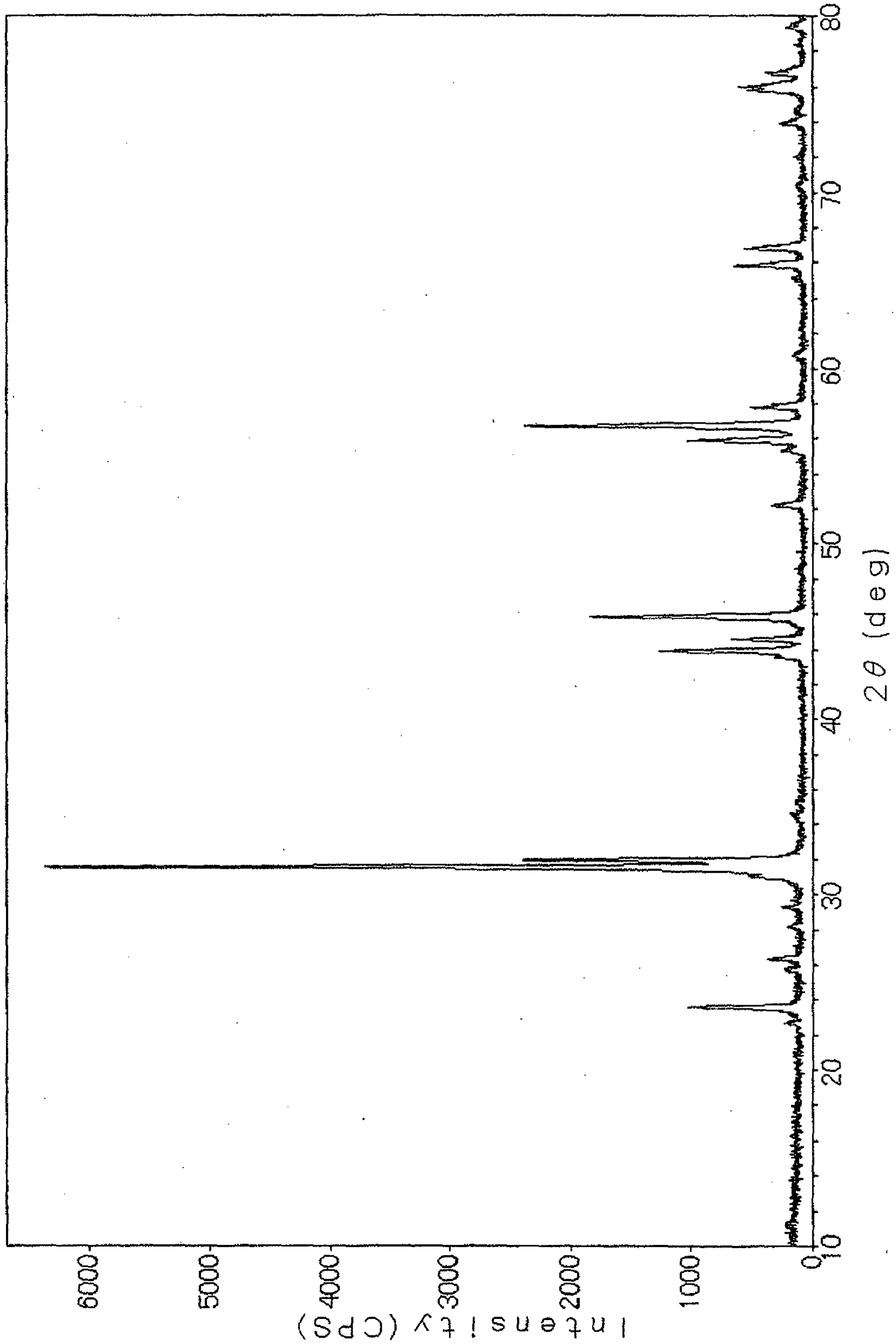

FIG. 5 $Nd_2Cu_{0.95}Pd_{0.05}O_4$

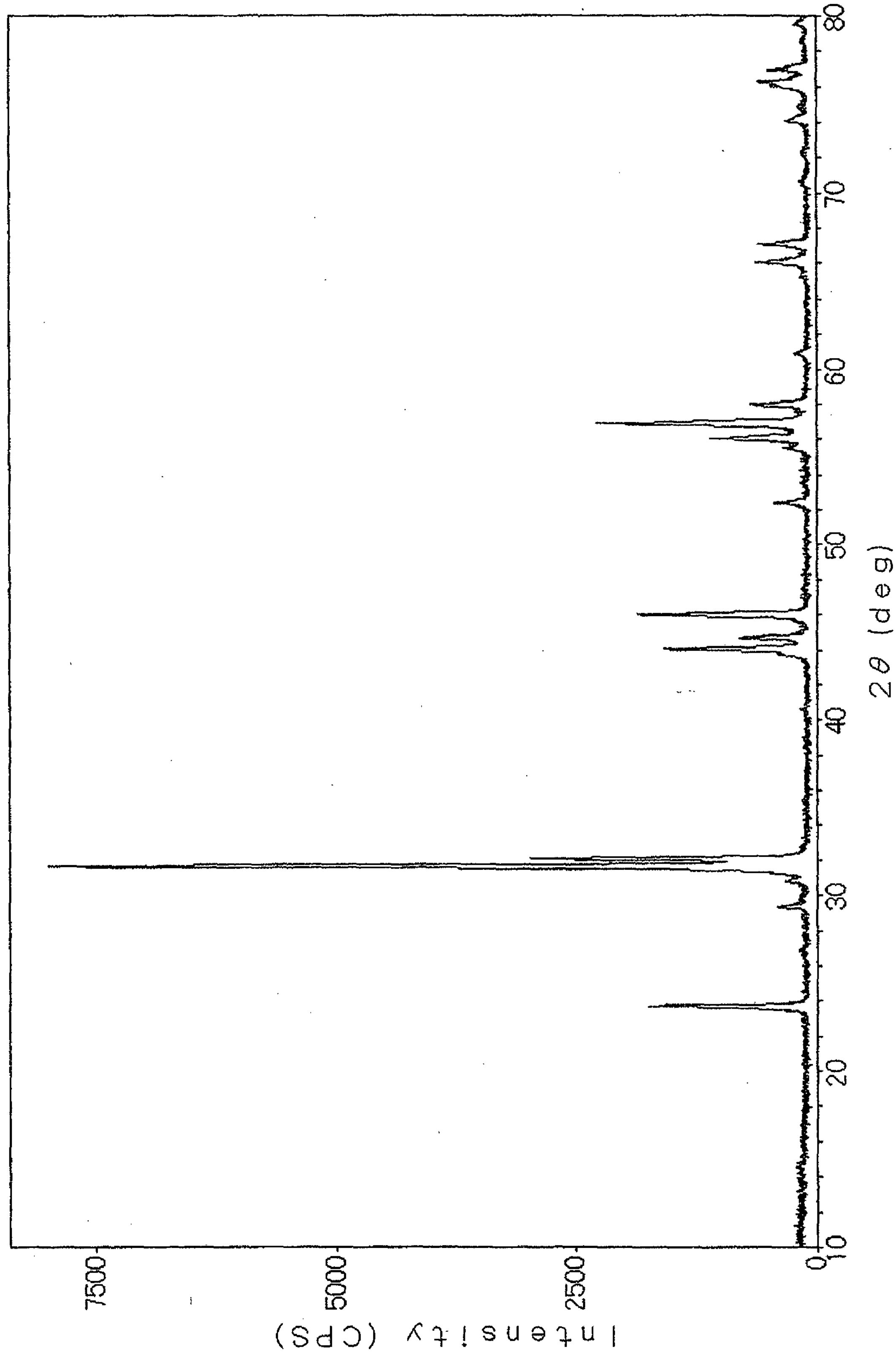

FIG. 6 $Nd_2Cu_{0.99}Pd_{0.01}O_4$

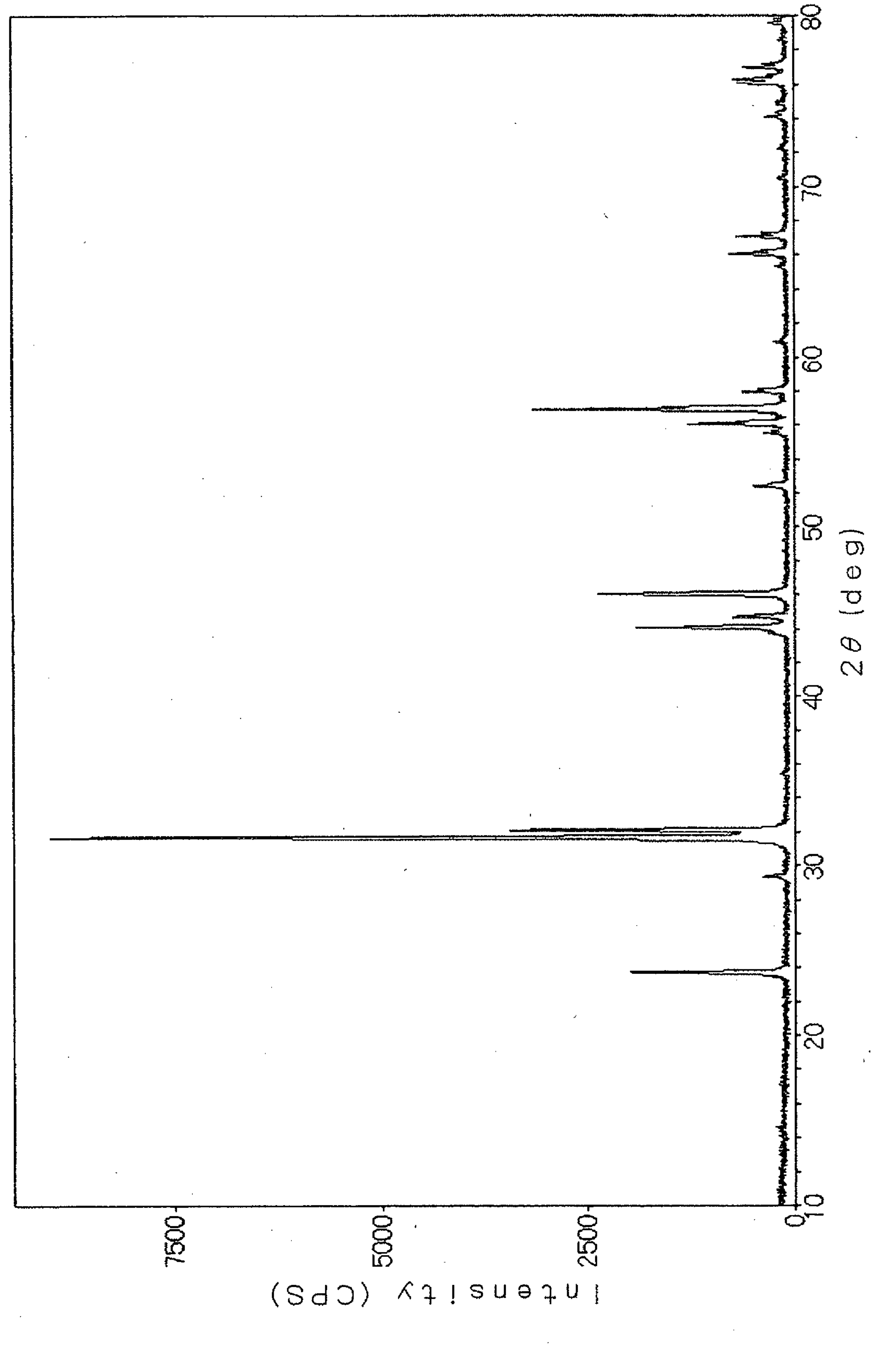

FIG. 7 $Nd_2Cu_{0.995}Pd_{0.005}O_4$

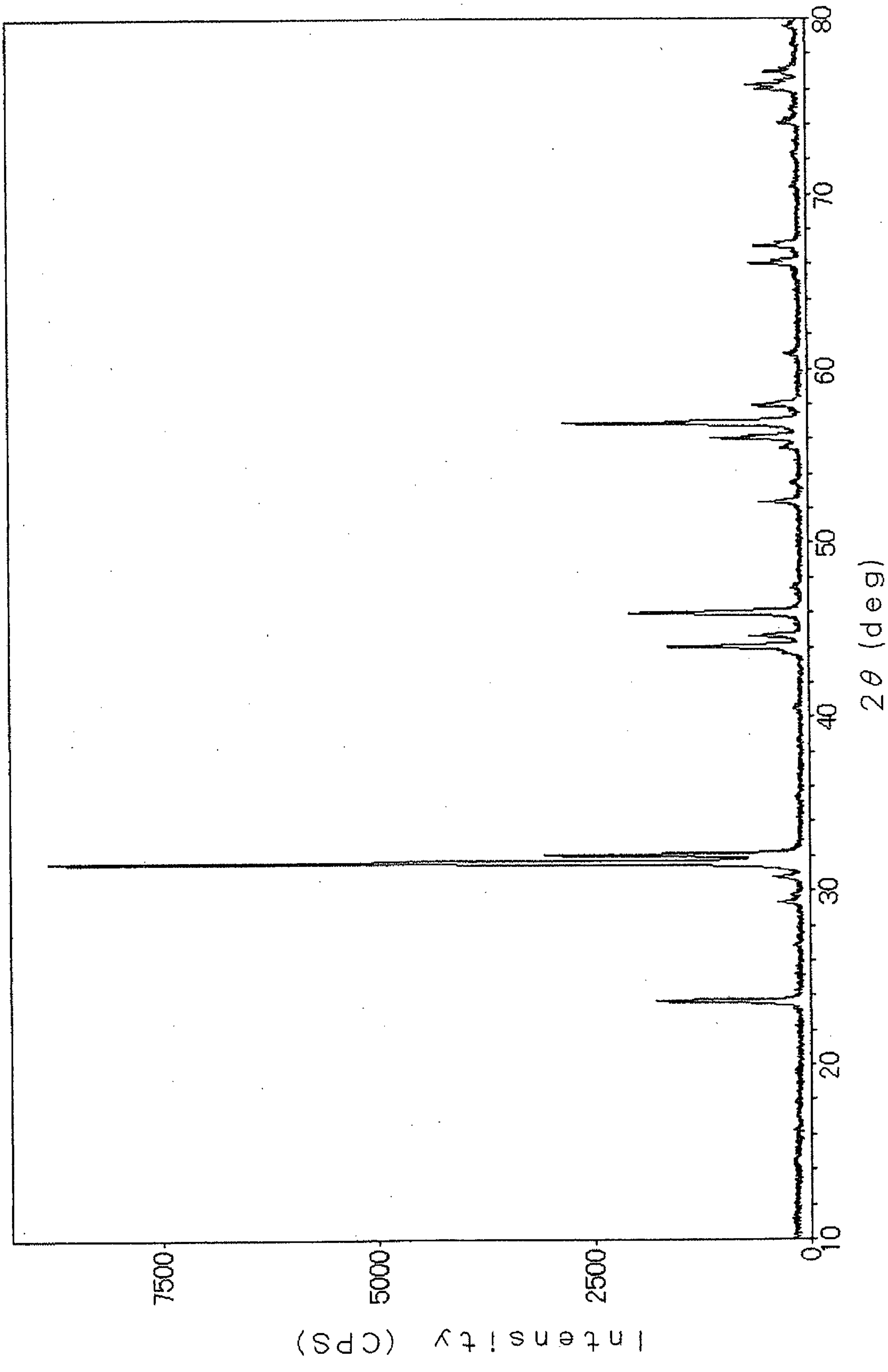

FIG. 8 $Nd_2Cu_{0.999}Pd_{0.001}O_4$

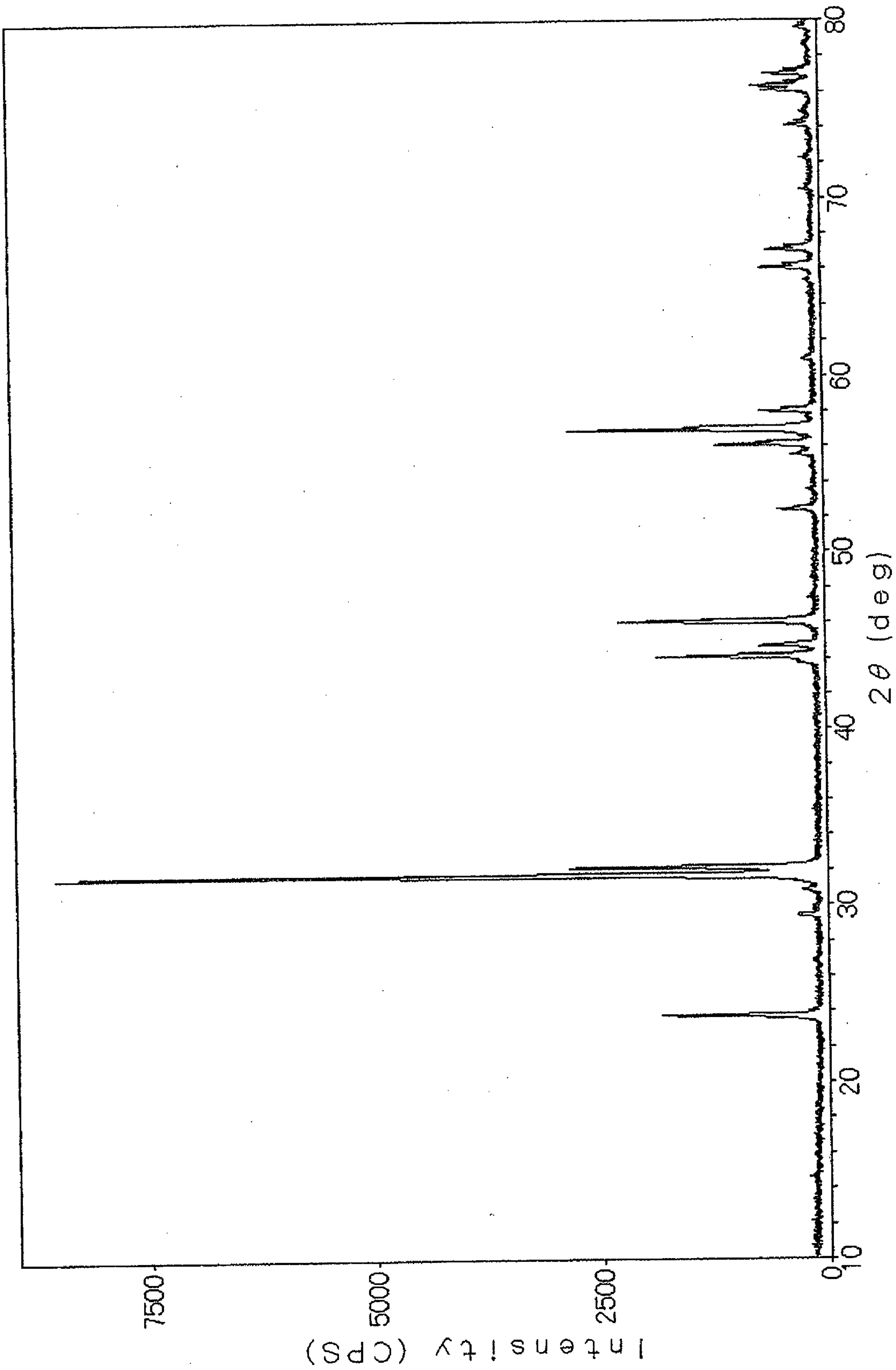

FIG. 9

$Gd_2Cu_{0.95}Pd_{0.05}O_4$

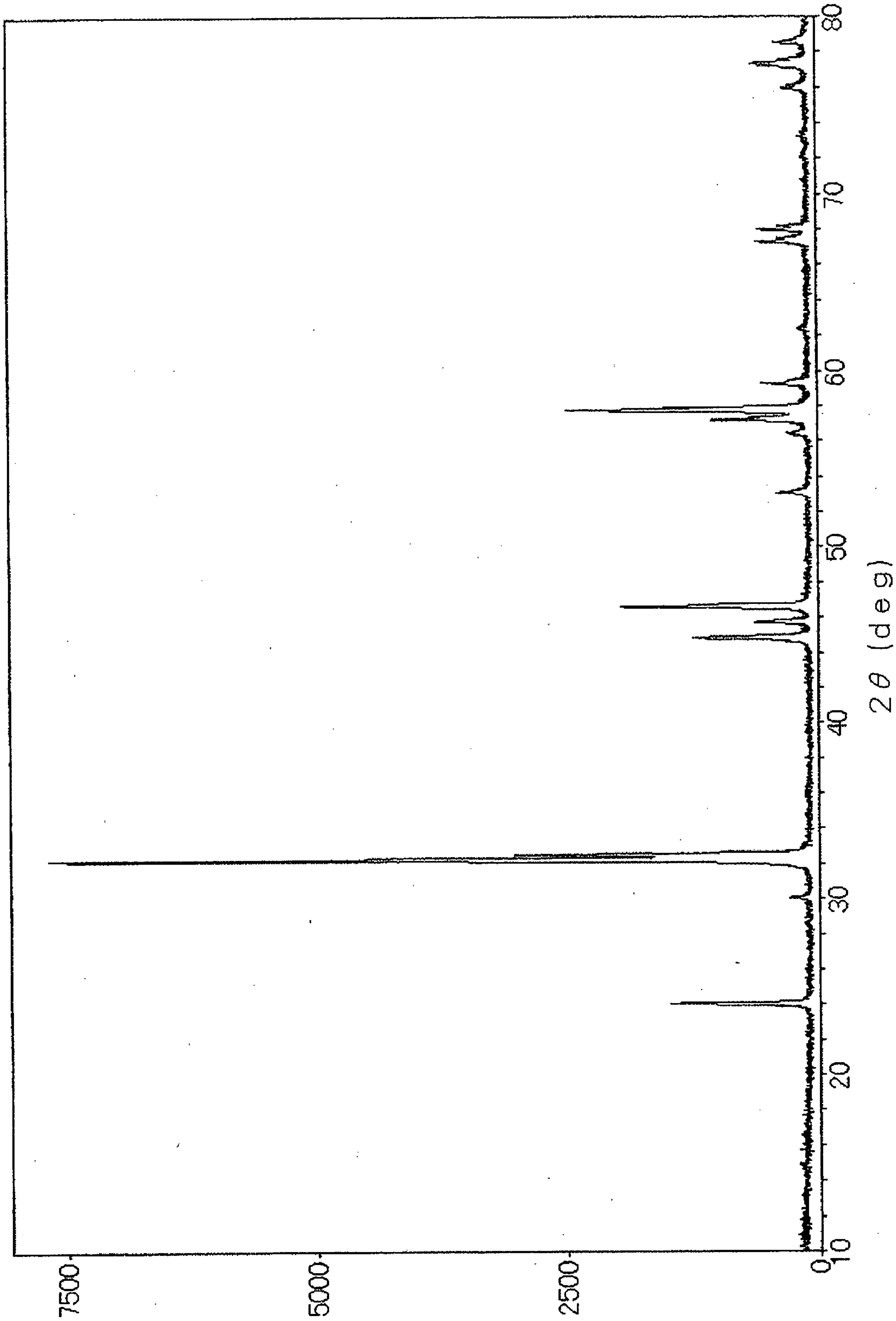

FIG.10 $Gd_2Cu_{0.94}Pd_{0.01}O_{3.95}$

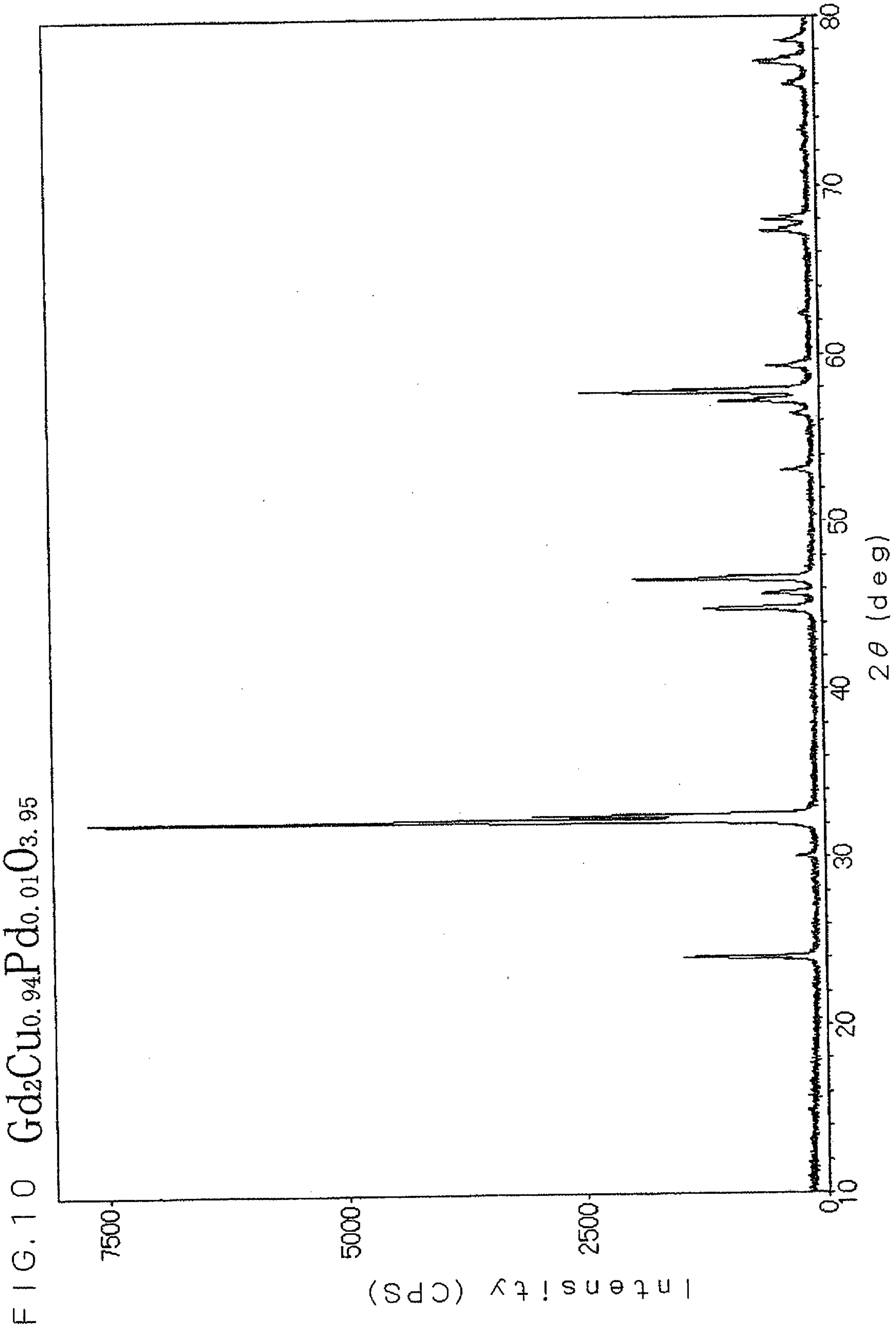

FIG. 11

$Gd_2Cu_{0.995}Pd_{0.005}O_4$

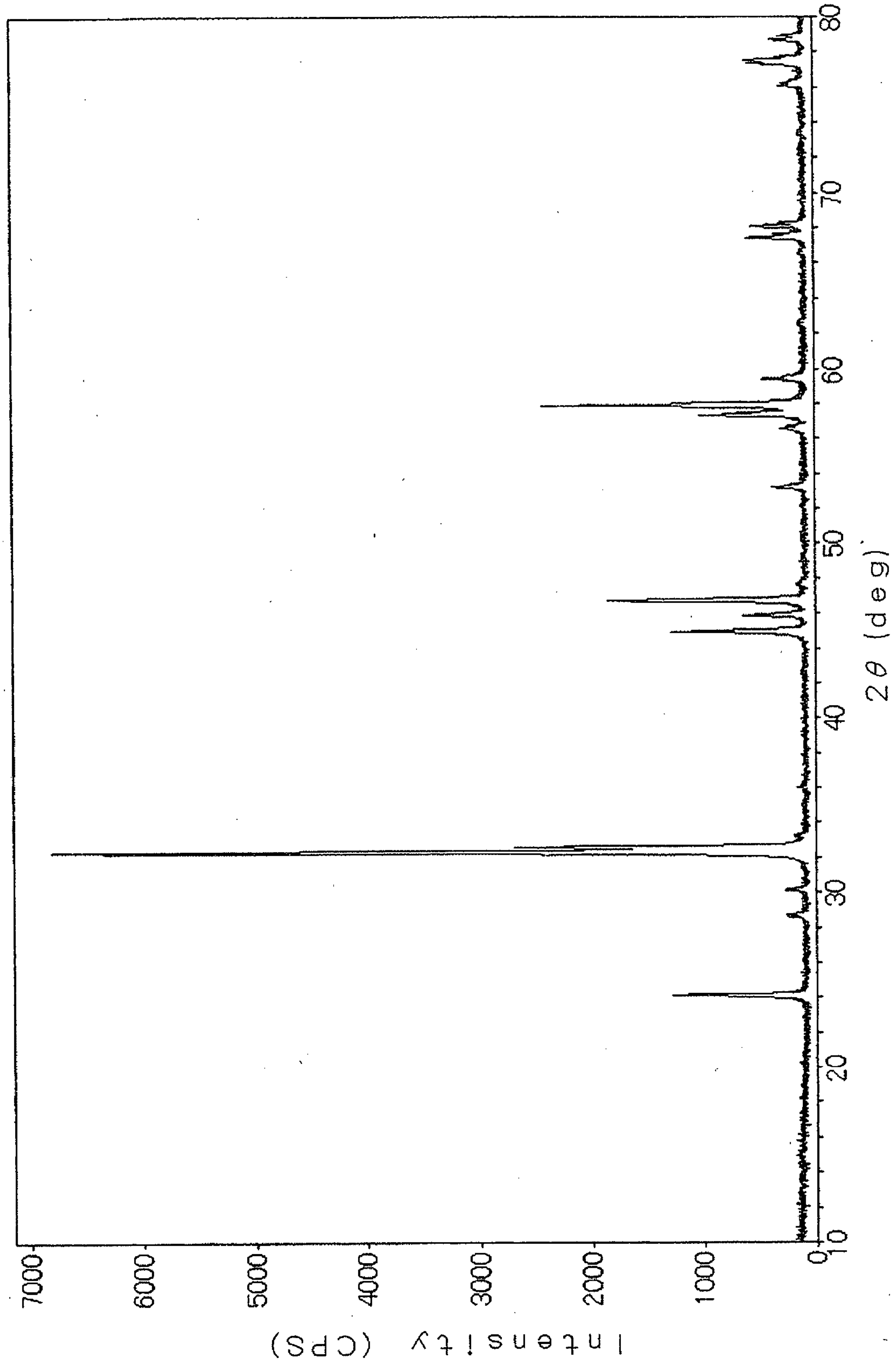

FIG. 12

$(La_{0.6}Sr_{0.4})_2Cu_{0.95}Pd_{0.05}O_4$

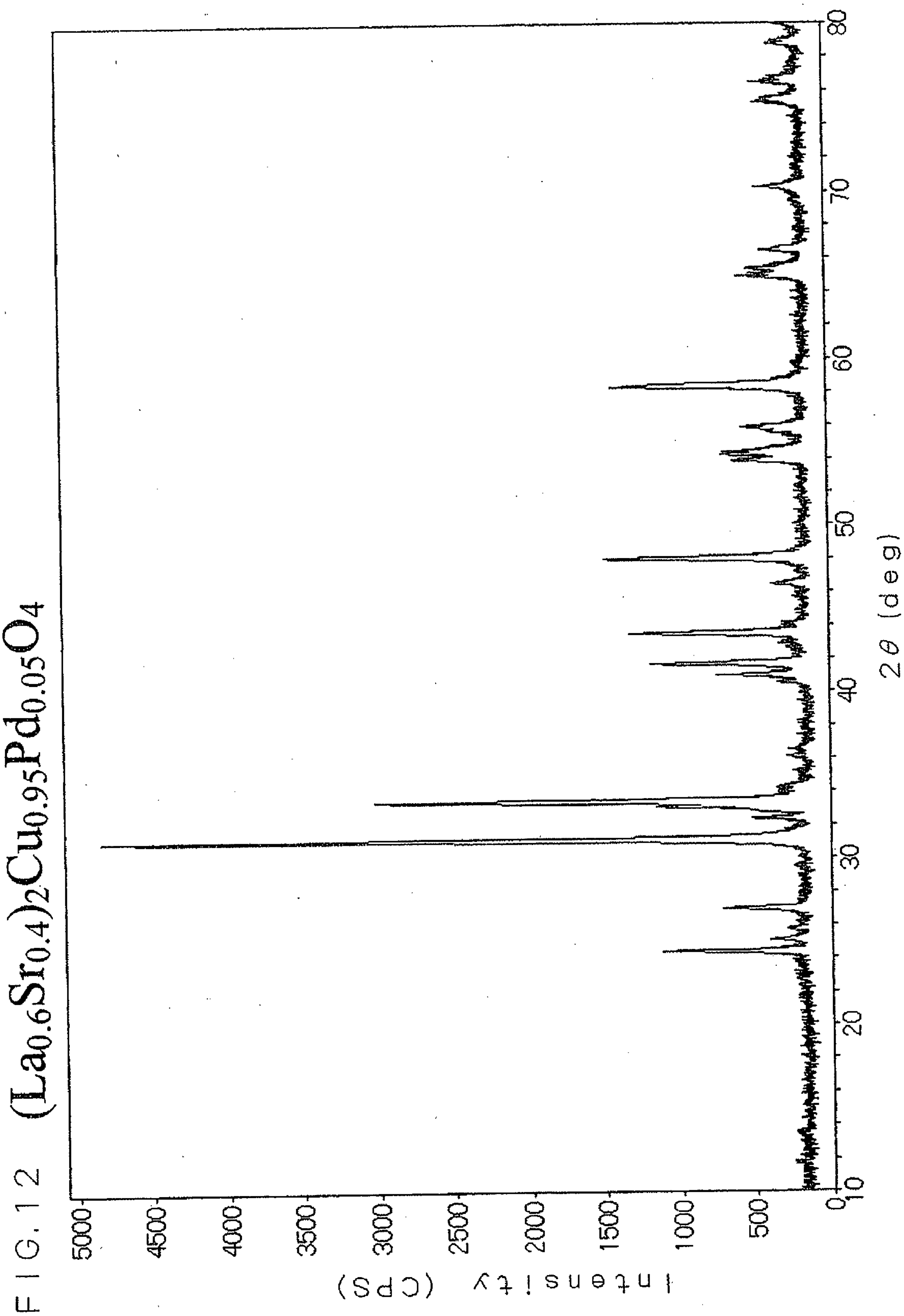

FIG. 13 $(La_{0.6}Sr_{0.4})_2Cu_{0.94}Pd_{0.01}O_{3.95}$

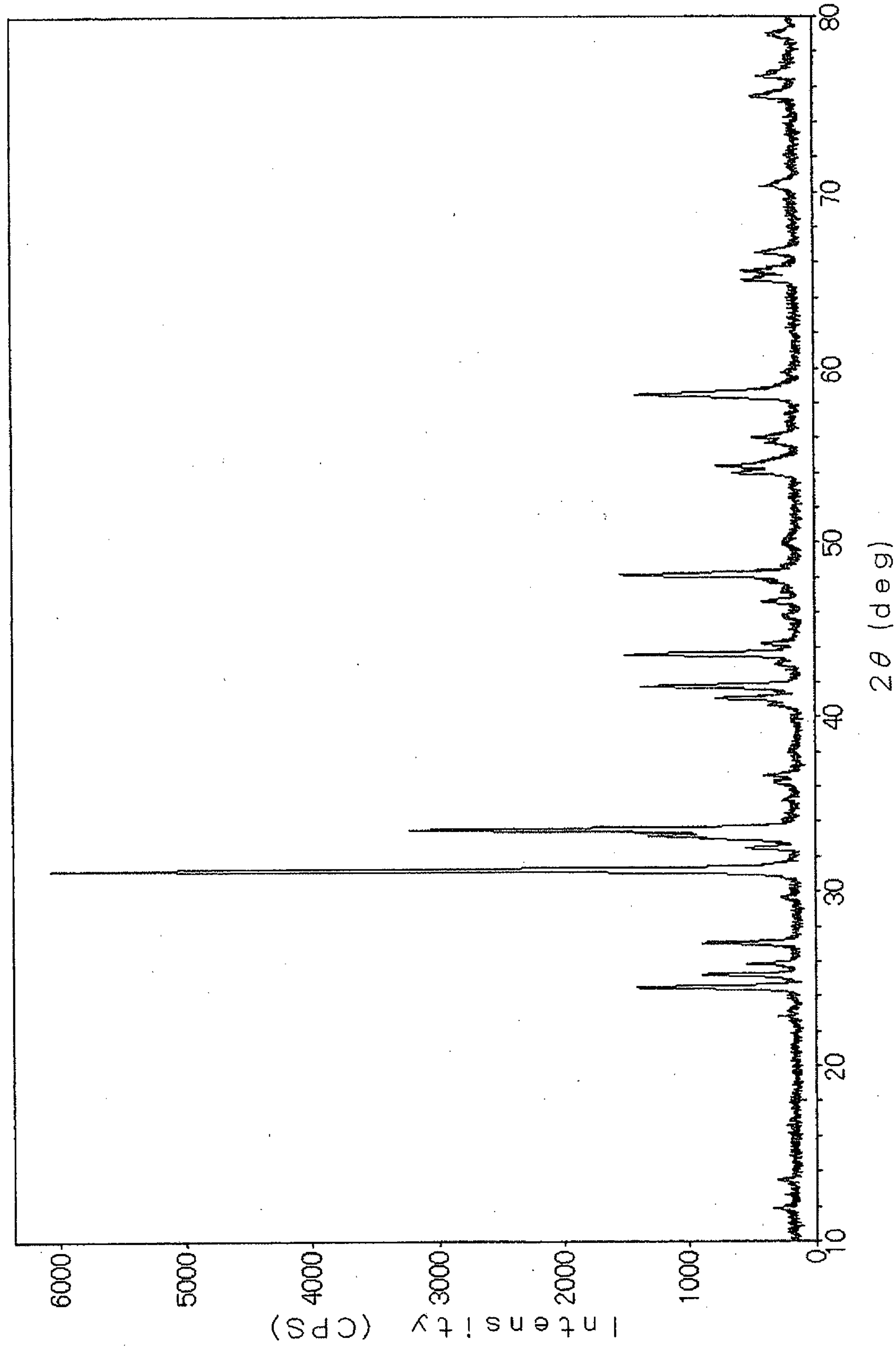

FIG. 14

$(Gd_{0.6}Sr_{0.4})_2Cu_{0.94}Pd_{0.01}O_{3.95}$

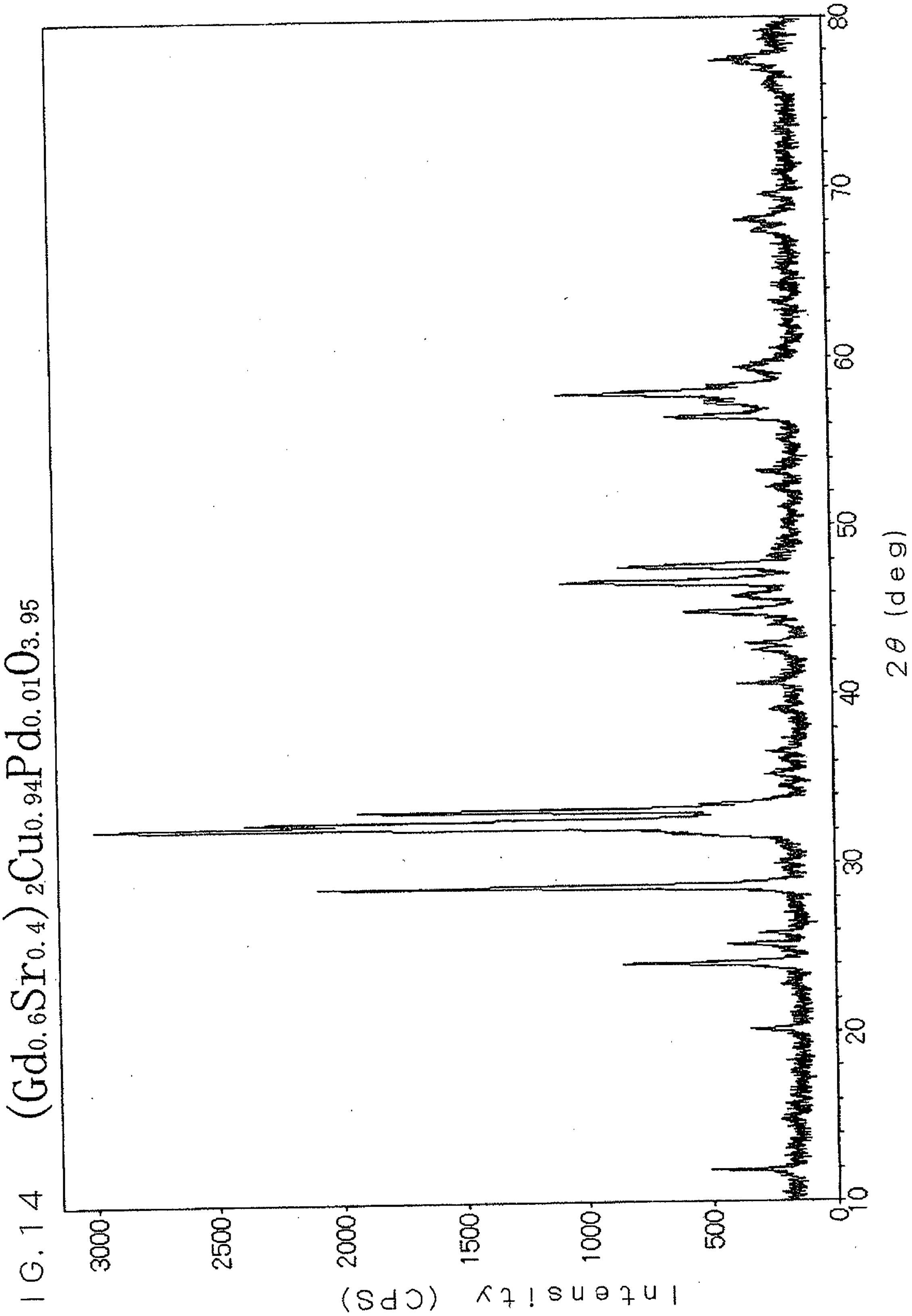

FIG. 15

$(Nd_{0.92}Ce_{0.08})_2Cu_{0.95}Pd_{0.05}O_4$

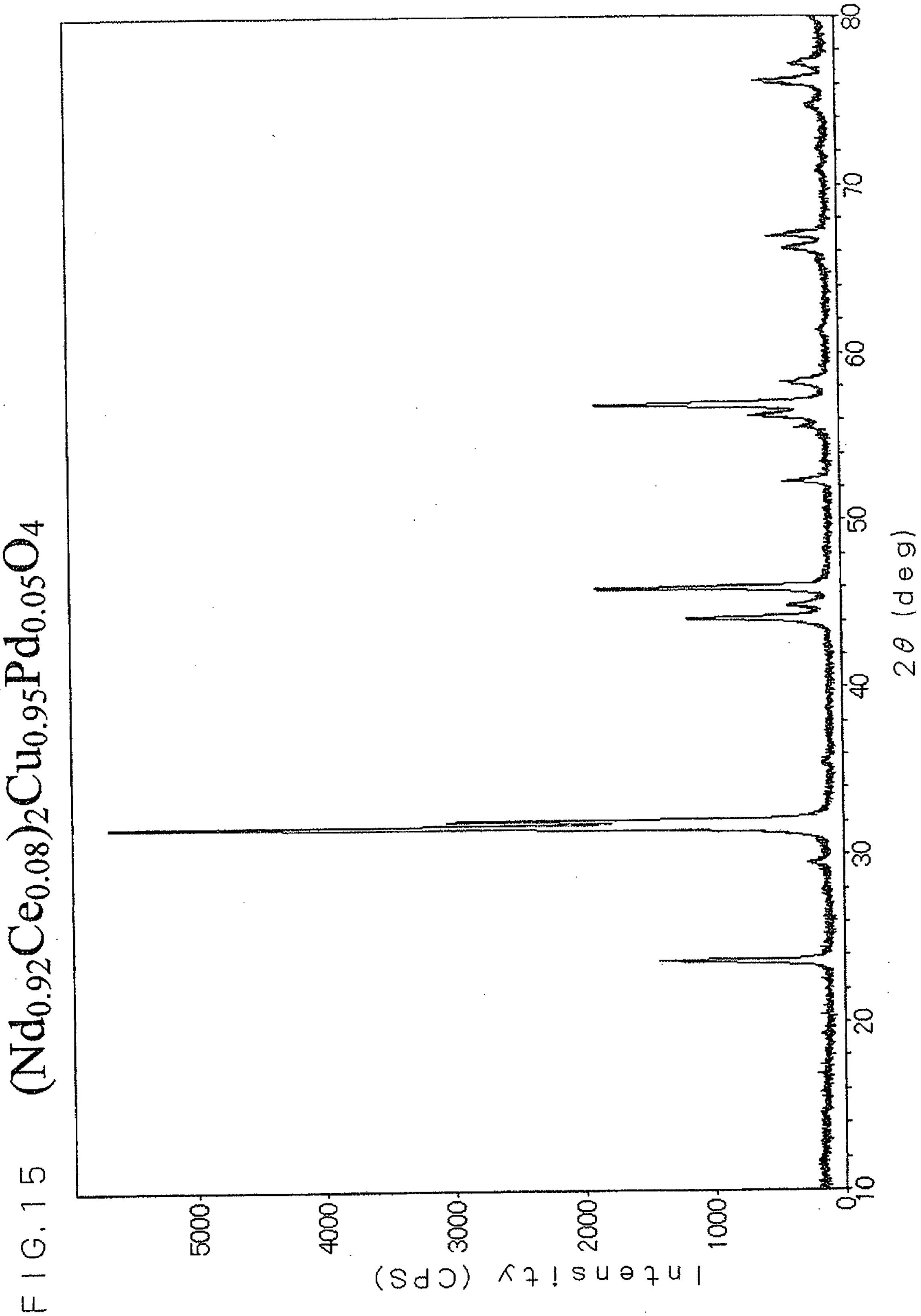

FIG. 16 $(La_{0.5}Y_{0.5})_2Cu_{0.95}Pd_{0.05}O_4$

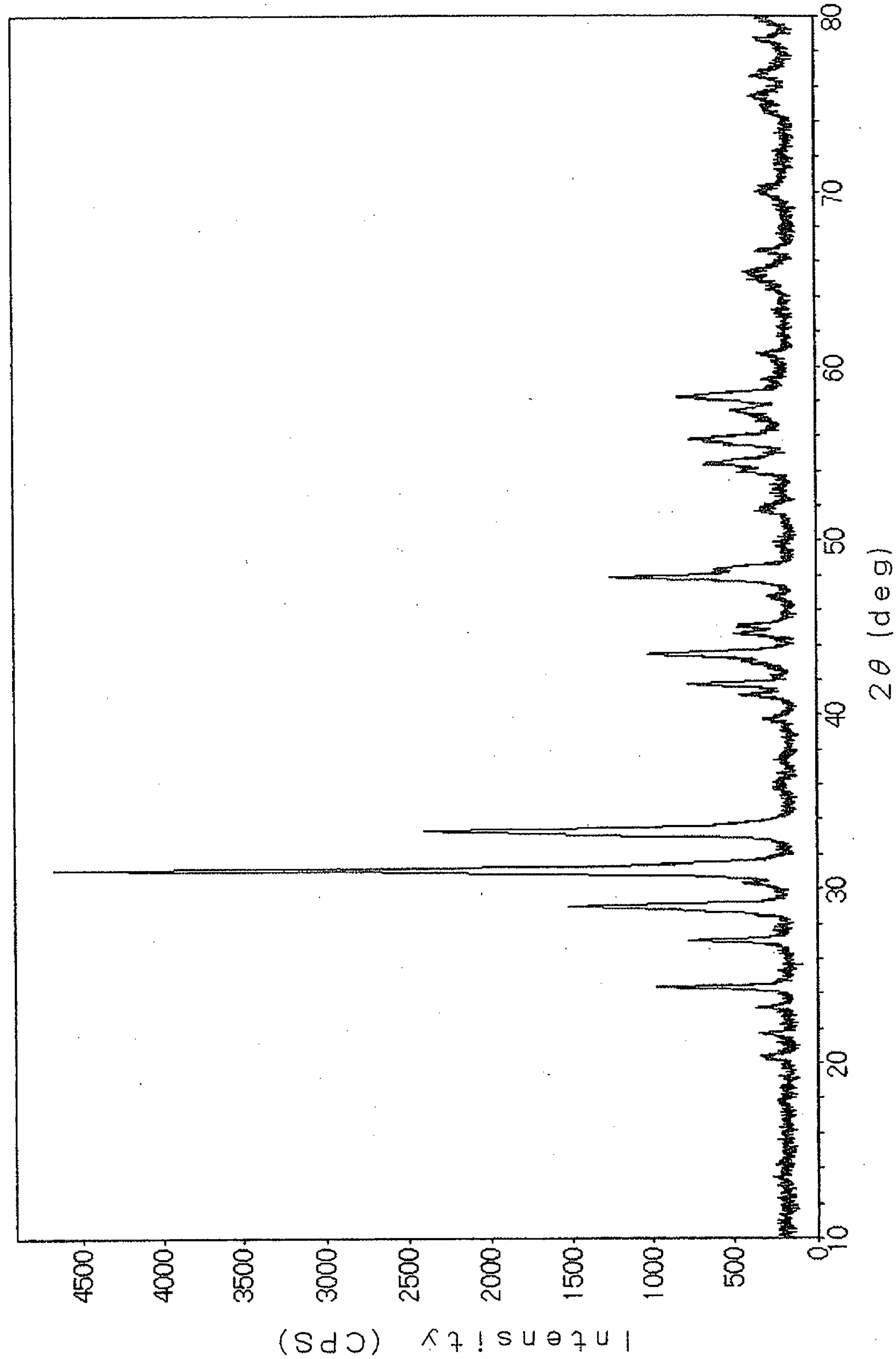

FIG. 17 $LaFe_{0.95}Pd_{0.05}O_3$

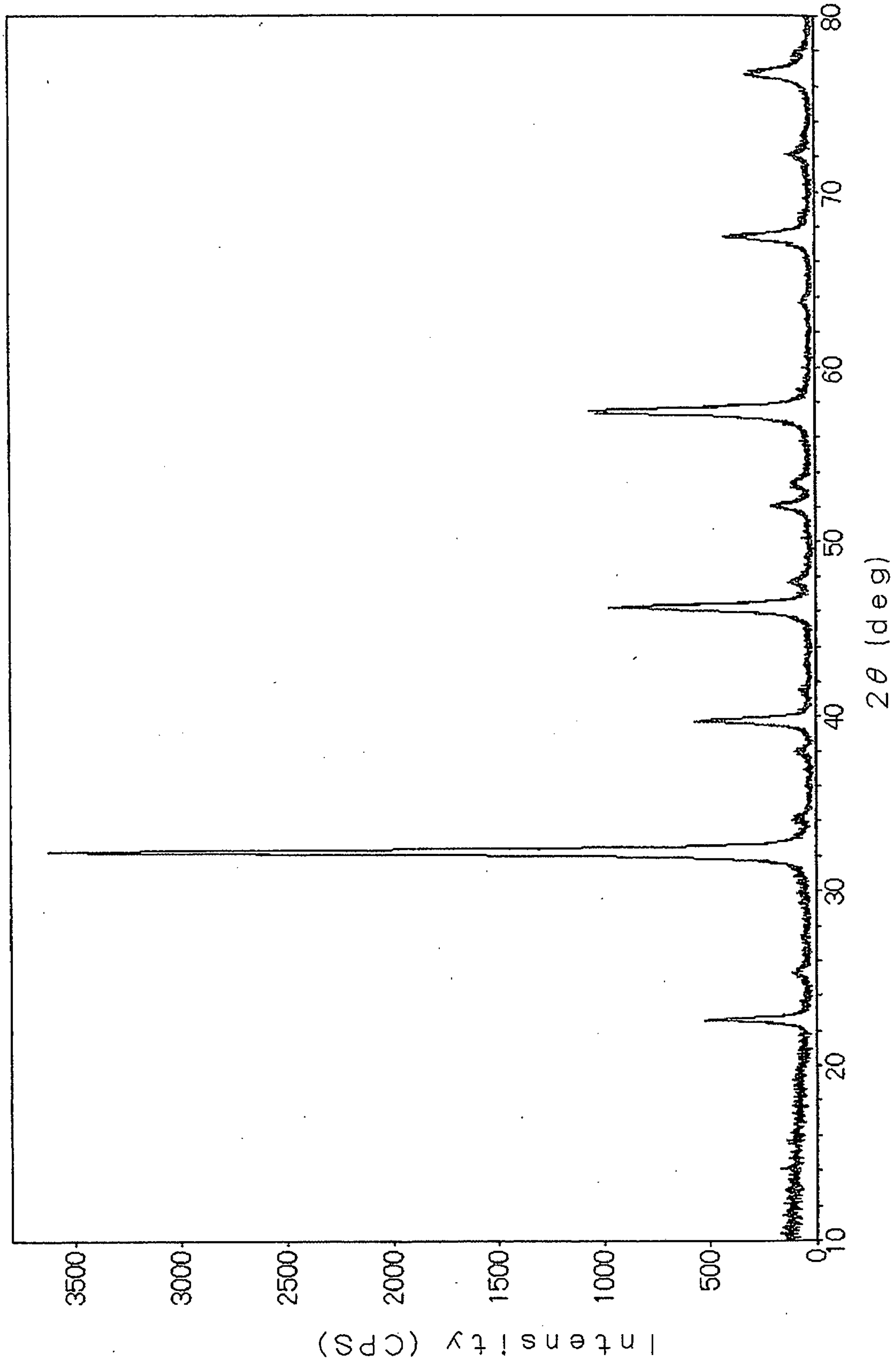

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2005314355 A
- US 20050215804 A1